Europäisches Patentamt
European Patent Office
Office européen des brevets

(11) Publication number: **0 424 538 A1**

(12) # EUROPEAN PATENT APPLICATION published in accordance with Art. 158(3) EPC

(21) Application number: **90904433.1**

(22) Date of filing: **09.03.90**

(86) International application number: **PCT/JP90/00313**

(87) International publication number: **WO 90/10622 (20.09.90 90/22)**

(51) Int. Cl.5: **C07D 213/16**, C07D 213/26, C07D 213/30, C07D 213/32, C07D 213/50, C07D 213/53, C07D 213/68, C07D 213/74, C07D 213/75, C07D 217/02, A01N 43/40

(30) Priority: **10.03.89 JP 58108/89**
**10.03.89 JP 58109/89**

(43) Date of publication of application: **02.05.91 Bulletin 91/18**

(84) Designated Contracting States: **BE CH DE FR GB IT LI NL SE**

(71) Applicant: **IDEMITSU KOSAN COMPANY LIMITED**
**1-1, Marunouchi 3-chome Chiyoda-ku**
**Tokyo 100(JP)**

(72) Inventor: **TERADA, Izumi Idemitsu Kosan Company Limited**
**1280, Kamiizumi Sodegaura-machi**
**Kimitsu-gun Chiba 299-02(JP)**
Inventor: **MATSUZAKI, Katsuhiko Idemitsu Kosan Company**
**Limited 1280, Kamiizumi Sodegaura-machi**
**Kimitsu-gun Chiba 299-02(JP)**
Inventor: **NONOSHITA, Kazuyoshi 21-14, Kurobegaoka**
**Hiratsuka-shi**
**Kanagawa 254(JP)**
Inventor: **FUJITA, Fumio 41-1, Kashiwacho**
**Asahi-ku Yokohama-shi**
**Kanagawa 241(JP)**

(74) Representative: **Türk, Gille, Hrabal**
**Brucknerstrasse 20**
**W-4000 Düsseldorf 13(DE)**

(54) **PYRIDINE DERIVATIVES AND THEIR SALTS, AND INSECTICIDAL/ACARICIDAL AGENT CONTAINING THE SAME AS ACTIVE INGREDIENT.**

(57) This invention relates to new pyridine derivatives represented by general formula (I) and their salts, and also an insecticidal/acaricidal agent containing the same as the active ingredient. In said formula, $R^1$ represents a $C_2$ to $C_{20}$ straight-chain, branched or cyclic alkyl, alkenyl or alkynyl group which may be substituted by a substituent containing halogen, oxygen, sulfur or nitrogen; $R^2$ represents a $C_1$ to $C_6$ straight-chain, branched or cyclic alkyl, alkenyl or alkynyl group, provided that $R^1$ has at least four carbon atoms in total, $R^1$ is different from $R^2$, and part of $R^1$ and $R^2$ are each methylene and together form a $C_4$ to $C_8$ cyclic structure; and $R^3$ represents hydrogen or a $C_1$ to $C_6$ straight-chain or branched alkyl group.

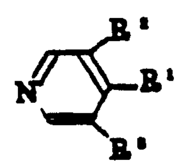

------- ( I )

EP 0 424 538 A1

# A PYRIDINE DERIVATIVE AND SALTS THEREOF AS WELL AS INSECTICIDAL-ACARICIDAL AGENT COMPRISING THE PYRIDINE DERIVATIVE OR SALT THEREOF AS THE EFFECTIVE INGREDIENT

## Field of technology

The present invention relates to a pyridine derivative and salts thereof having strong insecticidal-acaricidal activity as well as to an insecticidal-acaricidal agent comprising the said pyridine derivative or a salt thereof as the effective ingredient.

## Background technology

Various kinds of insecticidal-acaricidal agents are hitherto under use for the elimination of the pests on agricultural crops and horticultural crops, control of unsanitary pests and so on and typical ones include chlorine-based ones, organic phosphorus-based ones, carbamate-based ones, pyrethroid-based ones and the like.

These chemicals, however, are causing problems in safety such as retentiveness, accumulativeness and the like and problems of environmental pollution as well as problems of drug resistance and so on.

Accordingly, it is desired to develop a substance free from the above mentioned problems and still having strong insecticidal-acaricidal activity.

Known pyridine derivatives include, for example, those described below but each of them is not described at all relative to the application as an agricultural chemical.

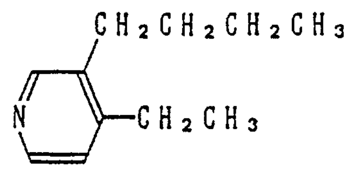

(Chemical Abstracts, 65, 10556f, 1966; Collection Czech. Chem. Commun., 31, 3008, 1966)

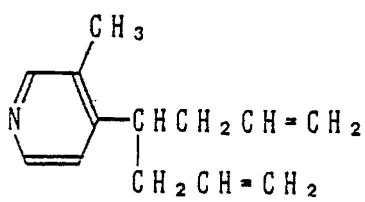

(Chemical Abstracts, 85, 32850y, 1976; Japanese Patent Kokai 50-129571)

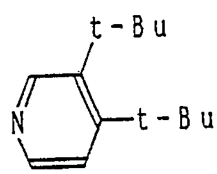

(Chemical Abstracts, 100, 155997n, 1984; J.O.C., 49, 1338, 1984)

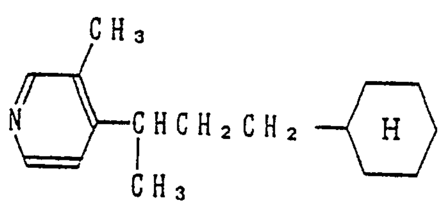

(Chemical Abstracts, 109, 6426j, 1988; EP 253681)

And, according to Chemical Abstracts, 109, 6426j, 1948 and Journal of Economic Entomology, 41, 98, 1948, 4-n-amyl pyridine and 4-(5-nonyl) pyridine were tested against cabbage maggot (Hylemya brassicae) as a kind of hanabae but no activity was recognized.

Disclosure of the invention

The present invention provides (1) a pyridine derivative represented by the general formula (I)

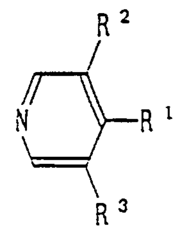

···· (I)

[in the formula, $R^1$ denotes a straight-chain or branched alkyl group of 2 to 20 carbons and the carbons of the alkyl chain in $R^1$ can be substituted with the following substituent groups at any position, in any number and in any combination where the direction of bonding is also non-limitative.

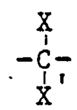

(in which
X is a halogen and denotes a fluorine, chlorine, bromine or iodine and X' denotes a hydrogen or a halogen),

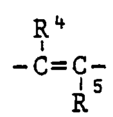

(in
which $R^4$ and $R^5$ each denote a hydrogen or an alkyl group of 1 to 4 carbons),

$-C\equiv C-$, $-O-$, $-S-$, 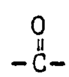, 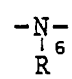

(in which $R^6$ denotes a hydrogen or an alkyl group of 1 to 4 carbons)

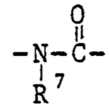

, (in
which $R^7$ denotes a hydrogen or an alkyl group of 1 to 4 carbons) and =N-O-. and, the hydrogen at the carbon terminal of $R^1$ can be replaced with the following substituent group. Cycloalkyl group of 3 to 16 carbons, cycloalkyl group of 3 to 16 carbons substituted by any number of alkyl, alkenyl, alkynyl groups of 1 to 6 carbons or halogens (incidentally, these cyclic rings can be a bicyclo ring or tricyclo ring), cycloalkenyl group of 3 to 16 carbons, cycloalkenyl group of 3 to 16 carbons substituted by any number of alkyl, alkenyl, alkynyl groups of 1 to 6 carbons or halogens (incidentally, these cyclic rings can be a bicyclo

ring or a tricyclo ring), cyclic ether and cyclic thioether (a plural number of ethers can be included), halogen or trihalomethyl group. $R^2$ denotes a straight-chain or branched alkyl group, alkenyl group or alkynyl group of 2 to 6 carbons, cycloalkyl group of 3 to 6 carbons or straight-chain or branched alkyl group, alkenyl group or alkynyl group of 1 to 6 carbons substituted with a cycloalkyl-group of 3 to 6 carbons. Incidentally, however, the total number of carbons in $R_1$ is at least 4 and $R^1$ and $R^2$ are not the same ones. And, a part of $R^1$ and $R^2$ each can be a methylene group forming a cyclic structure. In this case, the ring has a size of 4 to 8 carbons. $R^3$ denotes a hydrogen or a straight-chain or branched alkyl group of 1 to 6 carbons.] and a salt thereof, and (2) an insecticidal-acaricidal agent comprising, as the effective ingredient, a pyridine derivative represented by the general formula

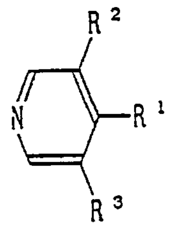

..... (I)

[in the formula, $R^1$ denotes a straight-chain or branched alkyl group of 2 to 20 carbons and the carbons of the alkyl chain in $R^1$ can be substituted with the following substituent groups at any position, in any number and in any combination where the direction of bonding is also non-limitative.

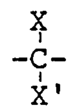

(in which X is a halogen and denotes a fluorine, chlorine, bromine or iodine and $X^1$ denotes a hydrogen or a halogen),

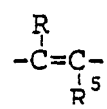

(in which $R^4$ and $R^5$ each denote a hydrogen or an alkyl group of 1 to 4 carbons)

$-C\equiv C-$, $-O-$, $-S-$, 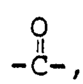, 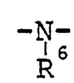

,
(in which $R^6$ denotes a hydrogen or an alkyl group of 1 to 4 carbons),

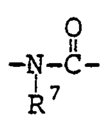

(in which $R^7$ denotes a hydrogen or an alkyl group of 1 to 4 carbons) and =N-O-. And, the hydrogen at the carbon terminal of $R^1$ can be replaced with the following substituent group. Cycloalkyl group of 3 to 16 carbons, cycloalkyl group of 3 to 16 carbons substituted by any number of alkyl, alkenyl, alkynyl groups of 1 to 6 carbons or halogens (incidentally, these cyclic rings can be a bicyclo ring or tricyclo ring), cycloalkenyl group of 3 to 16 carbons, cycloalkenyl group of 3 to 16 carbons substituted by any number of alkyl, alkenyl, alkynyl groups of 1 to 6 carbons or halogens (incidentally, these cyclic rings can be a bicyclo ring or tricyclo ring), cyclic ether and cyclic thioether (a plural number of ethers can be included), halogen

or trihalomethyl group. $R^2$ denotes a straight-chain or branched alkyl group, alkenyl group or alkynyl group of 2 to 6 carbons, cycloalkyl group of 3 to 6 carbons or straight-chain or branched alkyl group, alkenyl group or alkynyl group of 3 to 6 carbons. Incidentally, however, the total number of carbons in $R_1$ is at least 4 and $R^1$ and $R^2$ are not the same ones. And, a part of $R^1$ and $R^2$ each can be a methylene group forming a cyclic structure. In this case, the ring has a size of 4 to 8 carbons. $R^3$ denotes a hydrogen or a straight-chain or branched alkyl group of 1 to 6 carbons.] or a salt thereof.

Accordingly, the inventors have continued investigations in order to develop a substance having an insecticidal-acaricidal activity without causing the problems described above. As a result, they have arrived at a discovery that specific pyridine derivatives and salts thereof exhibit strong insecticidal-acaricidal activity leading to the present invention.

The novel pyridine derivative and a salt thereof according to the present invention exhibit strong insecticidal-acaricidal activity. Furthermore, there are caused no problems of retentiveness and accumulativeness because they are readily decomposable. And, the effect against pests is different from known chemicals due to the difference in the structure so that they can be used effectively even in the assistance of the pests of which a resistive species has already appeared. Accordingly, the present invention is useful for the elimination of the pests on agricultural crops, horticultural crops and the like, control of hygienic pests and so on.

## Best mode to practice the invention

The present invention provides a pyridine derivative represented by the general formula (I)

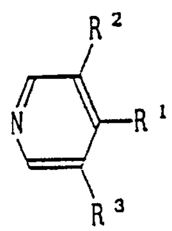

...... (I)

[in the formula, $R^1$ denotes a straight-chain or branched alkyl group of 2 to 20 carbons and the carbons of the alkyl chain in $R^1$ can be substituted with the following substituent groups at any position, in any number and in any combination where the direction of bonding is also non-limitative.

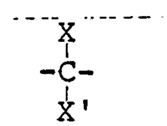

(in which X is a halogen and denotes a fluorine, chlorine, bromine or iodine and X' denotes a hydrogen or a halogen),

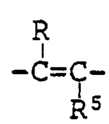

(in which $R^4$ and $R^5$ each denote a hydrogen or an alkyl group of 1 to 4 carbons),

$-C\equiv C-$, $-O-$, $-S-$, 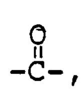, 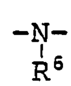

(in which $R^6$ denotes a hydrogen or an alkyl group of 1 to 4 carbons),

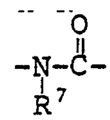

(in which $R^7$ denotes a hydrogen or an alkyl group of 1 to 4 carbons) and =N-O-. And, the hydrogen at the carbon terminal of $R^1$ can be replaced with the following substituent group. Cycloalkyl group of 3 to 16 carbons, cycloalkyl group of 3 to 16 carbons substituted by any number of alkyl, alkenyl, alkynyl groups of 1 to 6 carbons or halogens (incidentally, these cyclic rings can be a bicyclo ring or tricyclo ring), cycloalkenyl group of 3 to 16 carbons, cycloalkenyl group of 3 to 16 carbons substituted by any number of alkyl, alkenyl, alkynyl groups of 1 to 6 carbons or halogens (incidentally, these cyclic rings can be a bicyclo ring or tricyclo ring), cyclic ether and cyclic thioether (a plural number of ethers can be included), halogen or trihalomethyl group. $R^2$ denotes a straight-chain or branched alkyl group, alkenyl group or alkynyl group of 2 to 6 carbons, cycloalkyl group of 3 to 6 carbons or straight-chain or branched alkyl group, alkenyl group or alkynyl group of 3 to 6 carbons. Incidentally, however, the total number of carbons in $R_1$ is at least 4 and $R^1$ and $R^2$ are not the same ones. And, a part of $R^1$ and $R^2$ each can be a methylene group forming a cyclic structure. In this case, the ring has a size of 4 to 8 carbons. $R^3$ denotes a hydrogen or a straight-chain or branched alkyl group of 1 to 6 carbons.]or a salt thereof and also provides an insecticidal-acaricidal agent comprising a pyridine derivative represented by the above given general formula (I) or a salt thereof as the effective ingredient.

Particular examples of the pyridine derivative represented by the above given general formula (I) include those shown in the examples though not limited thereto. Incidentally, some of these pyridine derivatives have various kinds of stereoisomers (Z-isomers, E-isomers, R-isomers and S-isomers) and they are also included in the present invention.

The pyridine derivative represented by the above given general formula (I) can be prepared by various methods and examples thereof are shown below.

A halide compound represented by the general formula (II)

$$R^{1'}-X \qquad \cdots\cdots \quad (II)$$

(in the formula, $R^{1'}$ is a residue of the above mentioned $R^1$, $R^1$ is $-CHR^8R^{1'}$ ($R^8$ denotes a hydrogen or an alkyl group) and X denotes a halogen) is subjected to a condensation reaction with a substituted pyridine represented by the general formula (III)

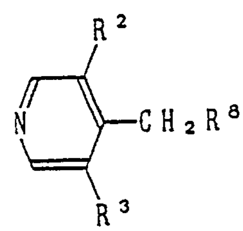

...... (III)

(in the formula, $R^2$, $R^3$ and $R^8$ are each the same as given above) in a solvent in the presence of a base so that the following pyridine derivative can be prepared.

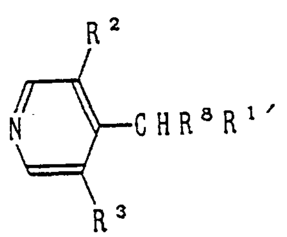

...... (I-1)

(in the formula $R^1$, $R^2$, $R^3$ and $R^8$ are each the same as given above)

The solvent used in this reaction includes aromatic hydrocarbons such as benzene, toluene and the like, ethers such as diethyl ether, tetrahydrofuran, dimethoxy ethane, diglyme and the like, polar aprotic solvents such as dimethyl formamide, dimethyl sulfoxide, hexamethyl phosphoric acid triamide and the like, liquid ammonia and so on.

And, lithium diisopropyl amine, tert-butoxy potassium, phenyl sodium, sodium amide and the like can be used as the base.

The reaction conditions can be adequately selected but it is usually preferable that the reaction temperature is -100 °C to +50 °C.

Further, metallic sodium or potassium is added to a substituted pyridine represented by the general formula (IV)

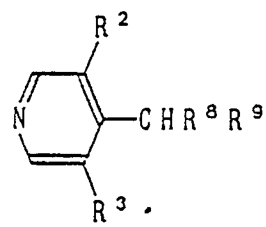

...... (IV)

(in the formula, $R^2$, $R^3$ and $R^8$ are each the same as given above and $R^9$ denotes a hydrogen or an alkyl group) to be completely reacted taking 3 to 5 hours to form a metal compound represented by the general formula (V)

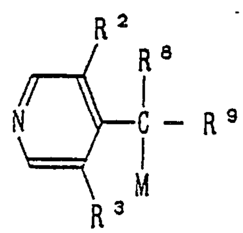

...... (V)

(in the formula, $R^2$, $R^3$, $R^8$ and $R^9$ are each the same as given above and M denotes a sodium or potassium), which is reacted with the compound represented by the general formula (VI)

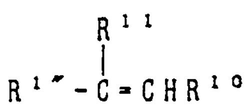

...... (VI)

(in the formula, $R^{1''}$ is a residue of $R^1$, $R^1$ is $R^{1''}$

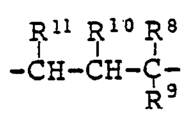

$R^8$ and $R^9$ are each the same as given above and $R^{10}$ and $R^{11}$ each denote a hydrogen or an alkyl group) so that the following pyridine derivative can be prepared.

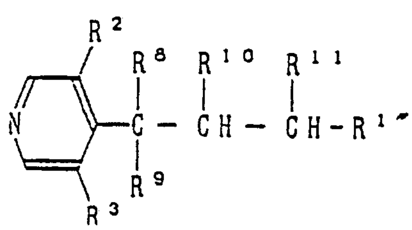

...... (I-2)

(in the formula, $R^{1''}$ $R^2$, $R^3$, $R^8$, $R^9$, $R^{10}$ and $R^{11}$ are each the same as given above)

This reaction rapidly proceeds at a temperature of 0 to 25 °C.

And, the halide compound represented by the above given general formula (II) is reacted in the presence of magnesium with the compound represented by the general formula (VII)

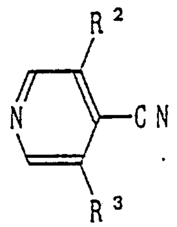

...... (VII)

(in the formula, $R^2$ and $R^3$ are each the same as given above) to form the compound represented by the general formula (VIII)

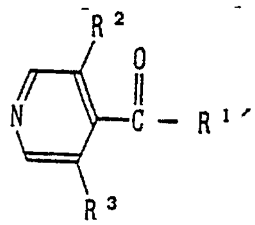

...... (VIII)

(in the formula, $R^{1'}$, $R^2$ and $R^3$ are each the same as given above) so that a carbonyl compound is obtained.

Following pyridine compound can be prepared by further reducing this compound by using hydrazine and an alkali metal hydroxide and the like.

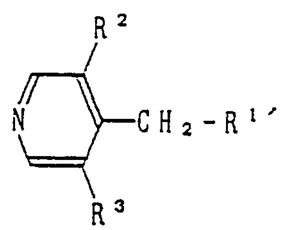

...... (I-3)

(in the formula, $R^{1'}$, $R^2$ and $R^3$ are each the same as given above)

It is preferable that this reaction is performed in a solvent such as ethylene glycol, diethylene glycol, triethylene glycol and the like. The other reaction conditions are not particularly limitative and can be adequately selected but the reaction temperature is preferably 180 to 220 °C.

Further, the pyridine derivative of the present invention represented by the general formula (I) can be prepared by the following steps (a) to (c) involving a Grignard reaction.

(a) The halide compound represented by the above given general formula (II) is reacted with magnesium in a solvent to prepare a Grignard reagent represented by the general formula (IX).

$$R^{1'}-MgX \quad \cdots\cdots \text{(IX)}$$

(in the formula, $R^{1'}$ is the same as given above and X denotes a halogen). The solvent used in this reaction is exemplified by ethers such as diethyl ether, tetrahydrofuran, dimethoxy ethane and the like. The other reaction conditions can be adequately selected but the reaction temperature is preferably 30 to 80 °C.

(b) The above mentioned Grignard reagent is reacted with the pyridyl ketone represented by the general formula (X)

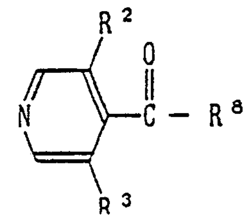

...... (X)

(in the formula, $R^2$, $R^3$ and $R^8$ are each the same as given above) to prepare an alcoholic compound represented by the general formula (XI)

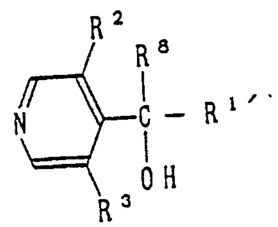

...... (XI)

(in the formula, $R^{1'}$, $R^2$, $R^3$ and $R^8$ are each the same as given above). This reaction is performed by using the same solvent as used in the above described step (a) at a temperature of -50 °C to +100 °C or, preferably, -10 °C to +20 °C. The other reaction conditions can be adequately selected.

(c) The alcoholic compound obtained in the above described step (b) is dehydrated by using a dehydrating agent in the presence or in the absence of a solvent to give the desired product (I-4) having a double bond introduced into the substituent group $R^1$.

In this reaction, an aromatic solvent such as benzene, toluene, xylene, pyridine and the like is used as the solvent and diluted sulfuric acid, concentrated sulfuric acid, diphosphorus pentoxide, thionyl chloride, phosphorus oxychloride, phosphorus trichlorlde, phosphorus pentachloride and the like can be used as the dehydrating agent. The dehydration reaction is performed usually at a temperature of -30 °C to +150 °c.

Further, the pyridine derivative of the present invention represented by the general formula (I) can be prepared also by the following method.

A compound represented by the general formula (XII)

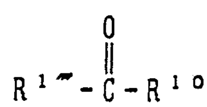

...... (XII)

(in the formula, $R^{1''}$ is a residue of $R^1$, $R^1$ is

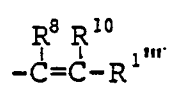

and $R^8$ and $R^{10}$ are each the same as given above) is subjected to an addition reaction in a solvent in the presence of a base with the substituted pyridine represented by the general formula (III)

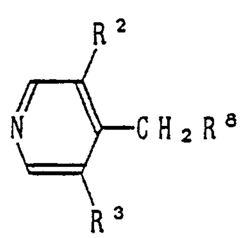

...... (III)

(in the formula, $R^2$, $R^3$ and $R^8$ are each the same as given above) to give an alcoholic compound represented by the general formula (XIII)

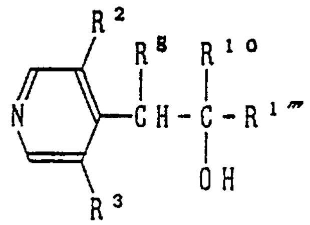

...... (XIII)

(in the formula, $R^{1'''}$, $R^2$, $R^3$, $R^8$ and $R^{10}$ are each the same as given above) and then this alcoholic compound is dehydrated so that the following pyridine derivative can be prepared.

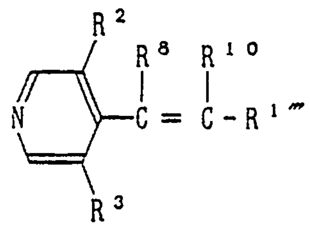

...... (I-5)

(in the formula, $R^{1'''}$ , $R^2$ , $R^3$ , $R^8$ and $R^{10}$ are each the same as given above)

The solvent used in the above mentioned addition reaction includes aromatic hydrocarbons such as benzene, toluene and the like, ethers such as diethyl ether, tetrahydrofuran, dimethoxy ethane, diglyme and the like, polar aprotic solvents such as dimethyl formamide, dimethyl sulfoxide, hexamethyl phosphoric acid triamide and the like, liquid ammonia and so on.

And, lithium diisopropyl amine, tert-butoxy potassium, phenyl sodium, sodium amide and the like can be used as the base.

The conditions of the above mentioned addition reaction are not particularly limitative but the reaction temperature should appropriately be -100 °C to +50 °C.

And, the dehydration reaction of the alcoholic compound is performed by using diluted sulfuric acid, concentrated sulfuric acid, diphosphorus pentoxide, thionyl chloride, phosphorus oxychloride, phosphorus trichloride, phosphorus pentachloride and the like and, although the reaction there can be performed without any solvent, aromatic solvents such as benzene, toluene, xylene, pyridine and the like can be used. The other conditions in the dehydration reaction can be adequately selected but the temperature is preferably in the range from -30 °C to +150 °C.

As to the pyridine derivative of the present invention represented by the general formula (I), an ester represented by the general formula (XIV)

$$R^{1''''}-COOR^{12} \qquad \cdots\cdots \quad (XIV)$$

(in the formula, $R^{1''''}$ is a residue of $R^1$, $R^1$ is

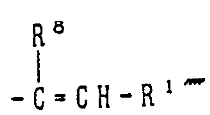

and $R^{12}$ denotes an alkyl group) is subjected to a condensation reaction in a solvent in the presence of a base with the substituted pyridine represented by the above given general formula (III) to give a ketone of the general formula (XV).

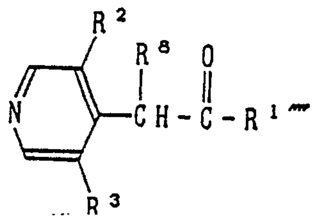

...... (XV)

(in the formula, $R^{1''''}$, $R^2$, $R^3$ and $R^8$ are each the same as given above)

The thus obtained ketone is reduced to form an alcoholic compound and this alcoholic compound is dehydrated to give the pyridine derivative represented by the following formula.

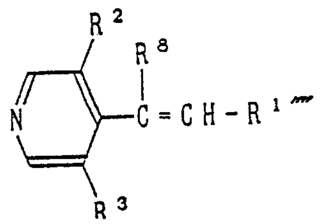

...... (I-6)

(in the formula, $R^{1''''}$, $R^2$, $R^3$ and $R^8$ are each the same as given above)

The solvent used in the above mentioned condensation reaction includes aromatic hydrocarbons such as benzene, toluene and the like, ethers such as diethyl ether, tetrahydrofuran, dimethoxy ethane, diglyme and the like, polar non-solvents such as dimethyl formamide, dimethyl sulfoxide, hexamethyl phosphoric acid triamide and the like, liquid ammonia and so on.

And, lithium diisopropyl amine, tert-butoxy potassium, phenyl sodium, sodium amide and the like can be used as the base. The conditions of the condensation reaction can be adequately selected but the reaction temperature is preferably -100 °C to +50 °C.

In the synthesis of the alcohol from the ketone, in the next place, sodium borohydride, sodium borocyano hydride, lithium aluminum hydride and the like are used as the catalyst. And, as the solvent, tetrahydrofuran, ether and the like are used for the former two and alcohols, hydrated alcohols and the like are preferred for the last one. The temperature of this reaction is preferably 0 to 70 °C or, in particular, 10 to 20 °C.

And, the dehydration reaction of the alcoholic compound is performed by using diluted sulfuric acid, concentrated sulfuric acid, diphosphorus pentoxide, thionyl chloride, phosphorus oxychloride, phosphorus trichloride, phosphorus pentachloride and the like and, although this reaction can be performed in the absence of any solvent, it can be performed in the presence of an aromatic solvent such as benzene, toluene, xylene, pyridine and the like. The other conditions in the dehydration reaction can be adequately selected but the temperature is preferably in the range from -30 °C to +50 °C.

Further, the pyridine derivative of the present invention can be prepared also by the following method.

A substituted epoxide represented by the general formula (XVI)

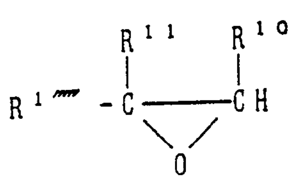

...... (XVI)

(in the formula, $R''''$ is a residue of $R^1$, $R^1$ is

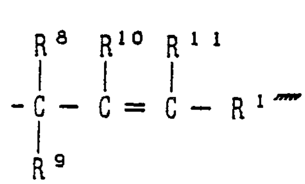

and $R^8$, $R^9$, $R^{10}$ and $R^{11}$ are each the same as given above) is subjected to an addition reaction in a solvent

in the presence of a base with the substituted pyridine represented by the above given general formula (IV) and the thus obtained alcoholic compound represented by the general formula (XVII)

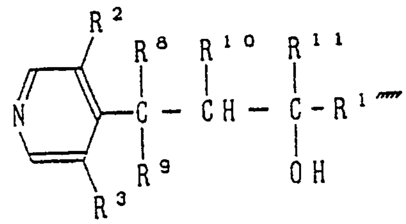

...... (XVII)

(in the formula, $R^{1'''''}$, $R^2$, $R^3$, $R^8$, $R^9$, $R^{10}$ and $R^{11}$ are each the same as given above) is dehydrated to give the pyridine derivative represented by the following formula.

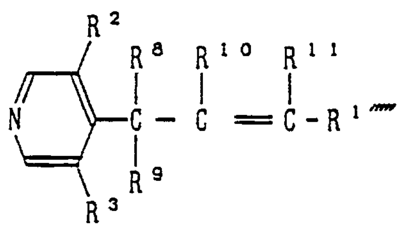

...... (I-7)

(in the formula, $R^{1'''''}$, $R^2$, $R^3$, $R^8$, $R^9$, $R^{10}$ and $R^{11}$ are each the same as given above)

The solvent used in the above mentioned addition reaction is exemplified by aromatic hydrocarbons such as benzene, toluene and the like, ethers such as diethyl ether, tetrahydrofuran, dimethoxy ethane, diglyme and the like, polar aprotic solvents such as dimethyl formamide, dimethyl sulfoxide, hexamethyl phosphoric acid triamide and the like, liquid ammonia and so on. Lithium diisopropyl amine, tert-butoxy potassium, phenyl sodium, sodium amide and the like can be used as the base. The conditions of the addition reaction can be adequately selected and the reaction temperature is preferably -100 °C to +50 °C.

And, the dehydration reaction of the alcohol is performed by using diluted sulfuric acid, concentrated sulfuric acid, diphosphorus pentoxide, thionyl chloride, phosphorus oxychloride, phosphorus trichloride, phosphorus pentachloride and the like and, although the reaction there can be performed without any solvent, it can be performed by using an aromatic solvent such as benzene, toluene, xylene, pyridine and the like. The other conditions in the dehydration reaction can be adeequately selected but the temperature is preferably -30 °C to +150 °c.

As to the pyridine derivative represented by the general formula

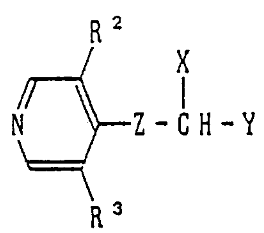

...... (I-8)

(in the formula, Y and Z each denote an alkyl residue in the above mentioned $R^1$ and X, $R^2$ and $R^3$ are each the same as given above), an alcoholic compound represented by the general formula

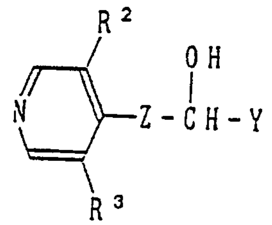

....... (XVIII)

(in the formula, Y, Z, $R^2$ and $R^3$ are each the same as given above) can be converted to the corresponding pyridine derivative represented by the general formula (I-8) by using a halogenating reagent. The halogenating reagent usable here includes phosphorus oxychloride, phosphorus trichloride, phosphorus pentachloride, carbon tetrachloride-triphenyl phosphine, hydrogen chloride, thionyl chloride, phosphorus tribromide, phosphorus pentabromide, bromine-triphenyl phosphine, carbon tetrabromide-triphenyl phosphine, thionyl bromide, dimethyl bromosulfonium bromide, hydrofluoric acid, diethylamino sulfate trifluoride, fluoroalkyl amines (for example, hexafluoropropene diethyl amine) and the like. Further, although the reaction can proceed without solvents, aromatic hydrocarbons such as benzene, toluene and the like, petroleum ether, acetonitrile, pyridine, DMF, dichloromethane, chloroform and the like can be used when a solvent is to be used. The reaction temperature depends on the halogenating reagent used and it is preferably from -70 °C to +30 °C in the case of fluorination and from -30 °C to +120 °C in the case of chlorination and bromination.

And, the alcoholic compound represented by the general formula (XVIII) is converted to a sulfonic acid ester followed by the reaction with an alkali halide so as to give the pyridine derivative represented by the general formula (I-8). The sulfonic acid ester here includes p-toluene sulfonic acid esters, methane sulfonic acid esters, trifluoromethane sulfonic acid esters and the like. The alkali halide includes lithium chloride, lithium bromide, magnesium bromide, calcium bromide, potassium bromide, potassium fluoride, cesium fluoride and the like. The solvent used in this reaction includes ethers such as ethyl ether, dimethoxy ethane, diethylene glycol dimethyl ether and the like, alcohols such as ethanol, isopropanol, diethylene glycol and the like, polar aprotic solvents such as dimethyl sulfoxide, dimethyl formamide and the like, and so on. The reaction temperature is preferably 0 °C to 180 °c.

The pyridine derivative represented by the general formula

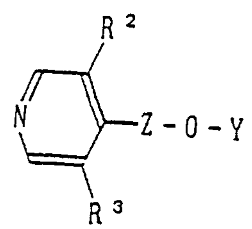

...... (I-9)

(in the formula, Y, Z, $R^2$ and $R^3$ are each the same as given above) can be obtained by the reaction of a compound represented by the general formula

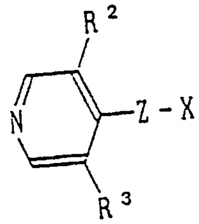

...... (XIX)

(in the formula, X, Z, $R^2$ and $R^3$ are each the same as given above) and a compound represented by the general formula

Y-O-M ...... (XX)

(in the formula, Y is the same as given above and M is an alkali metal). Here, chlorine, bromine, iodine and the like are used as the halogen of X and sodium, potassium and the like are used as the alkali metal of M. The solvent used in the reaction is exemplified by the polar aprotic solvents such as dimethyl formamide, dimethyl sulfoxide and the like and alcohols corresponding to the Y in the general formula (XX). And, the general formula (XX) can be prepared by the admixture of the corresponding alcohol compound with an alkali metal or an alkali metal hydride such as sodium hydride, potassium hydride and the like. The reaction temperature is preferably 20 °C to 150 °C.

Further, the pyridine derivative represented by the general formula (I-9) can be prepared also by the reaction of an alkylating agent represented by the general formula

Y-X" ...... (XXI)

(in the formula, Y is the same as given above and X" denotes a halogen or a sulfonic acid ester) and a compound represented by the general formula

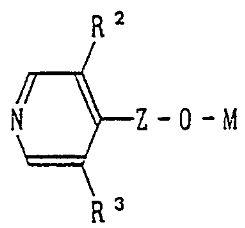

...... (XXII)

(in the formula, M and Z are each the same as given above). p-Toluene sulfonic acid esters, methane sulfonic acid esters, trifluoromethane sulfonic acid esters and the like are used here as the sulfonic acid ester of X". The compound represented by the general formula (XXII) can be prepared by the addition of an alkali metal hydride to a corresponding alcohol. The solvent used in the reaction is exemplified by the polar aprotic solvents such as dimethyl formamide, dimethyl sulfoxide and the like. The reaction temperature is preferably 20 °C to 150 °C.

Further, the pyridine derivative represented by the general formula

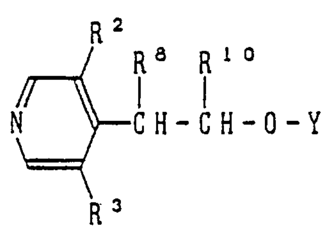

...... (I-9')

(in the formula, $R^2$, $R^3$, $R^8$, $R^{10}$ and Y are each the same as given above) can be prepared by the reaction of the compound represented by the above given general formula (XX) and a compound represented by the general formula

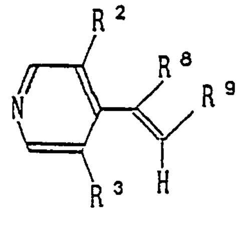

...... (XXIII)

(in the formula, $R^2$, $R^3$, $R^8$ and $R^{9'}$ are each the same as given above). The solvent used in the reaction includes the alcohols corresponding to the Y in the general formula (XX).

The pyridine derivative represented by the general formula

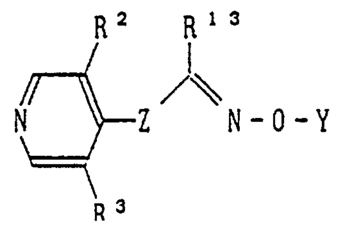

...... (I-10)

(in the formula, $R^2$, $R^3$, Z and Y are each the same as given above and $R^{13}$ denotes a hydrogen or an alkyl group) can be prepared by the reaction of the alkylating agent represented by the above given general formula (XXI) and an oxime derivative represented by the general formula

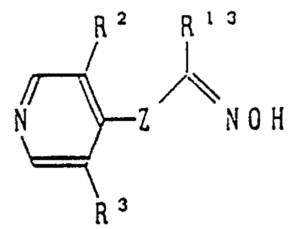

...... (XXIV)

(in the formula, $R^2$, $R^3$, $R^{13}$ and Z are each the same as given above) in the presence of a base under a solvent. As the base used in the reaction, metal hydrides such as sodium hydride, potassium hydride and the like, metal hydroxides such as sodium hydroxide, potassium hydroxide. and the like, sodium amide and the like are used. The solvent used in the reaction is exemplified by alcohols such as methanol, ethanol, isopropanol and the like, ethers such as ethyl ether, tetrahydrofuran, diethylene glycol dimethyl ether and the like, aromatic hydrocarbons such as benzene, toluene and the like, polar aprotic solvents such as dimethyl formamide, dimethyl sulfoxide and the like, liquid ammonia and so on. The reaction temperature is preferably -50 °C to +120 °C.

Further, the pyridine derivative represented by the general formula (I-10) can be obtained by the reaction of a compound represented by the general formula

$$Y\text{-}O\text{-}NH_2 \quad \cdots\cdots \quad (XXV)$$

(in the formula, Y is the same as given above) and a compound represented by the general formula

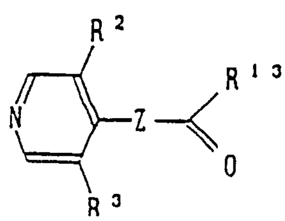

...... (XXVI)

(in the formula, $R^2$, $R^3$, $R^{13}$ and Z are each the same as given above). This reaction proceeds either under an acidic condition or under an alkaline condition. When performed under an acidic condition, hydrochloric acid, hydrobromic acid, diluted sulfuric acid and the like are used and water or a hydrated alcohol is used as the solvent. When performed under an alkaline condition, metal hydroxides such as sodium hydroxide, potassium hydroxide and the like, carbonates such as sodium carbonate, potassium carbonate and the like, carboxylic acid salts such as sodium acetate and the like and so on are used as the base and water or a hydrated alcohol is used as the solvent. The reaction temperature is preferably 20 °C to 100 °C.

Further, the pyridine derivative represented by the general formula

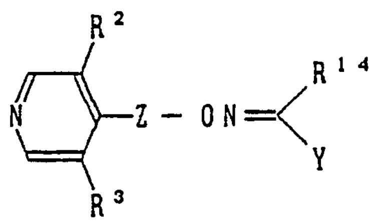

...... (I-10')

(in the formula, $R^2$, $R^3$, Y and Z are each the same as given above and $R^{14}$ denotes a hydrogen or an alkyl group) can be prepared by the reaction of a compound represented by the general formula

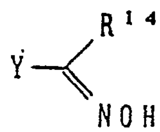

...... (XXVII)

(in the formula, Y and $R^{14}$ are each the same as given above) and a compound represented by the general formula

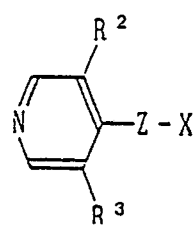

...... (XXVIII)

(in the formula, $R^2$, $R^3$, X and Z are each the same as given above). The reaction conditions in this case are the same as in the case of the reaction of the alkylating agent represented by the above given general formula (XXI) and the compound represented by the general formula (XXIV) in the presence of a base.

The pyridine derivative represented by the general formula

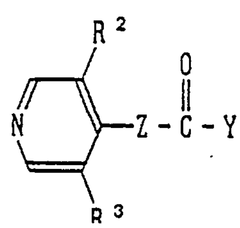

...... (I-11)

(in the formula, $R^2$, $R^3$, Y and Z are each the same as given above) can be prepared by the reaction of a nitrile represented by the general formula

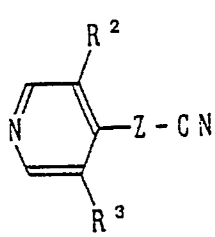

...... (XXIX)

(in the formula, $R^2$, $R^3$ and Z are each the same as given above) with a Grignard reagent represented by the general formula

Y-MgX ...... (XXX)

(in the formula, Y and X are each the same as given above) in a solvent followed by hydrolysis with an acid. The solvent used in the reaction is exemplified by ethers such as ethyl ether, tetrahydrofuran and the like and aromatic hydrocarbons such as benzene, toluene and the like. The reaction temperature is preferably 0 °C to 100 °C.

The pyridine derivative represented by the general formula

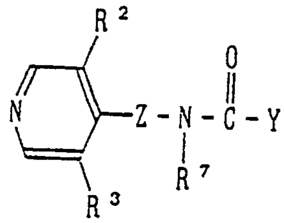

...... (I-12)

(in the formula, $R^2$, $R^3$, $R^7$, Y and Z are each the same as given above) can be prepared by the reaction of an amine represented by the general formula

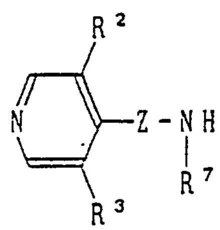

...... (XXXI)

(in the formula, $R^2$, $R^3$, $R^7$ and Z are each the same as given above) and a carboxylic acid derivative represented by the general formula

$(Y\text{-}CO)_2O$ ...... (XXXII)

(in the formula, Y is the same as given above). The reaction proceeds even without using a base but, when a base is to be used, organic bases such as pyridine, alkyl amines (triethyl amine, tributyl amine and the like), aryl amines (N,N-dimethyl aniline and the like) and the like or inorganic bases such as sodium hydroxide, potassium carbonate and the like can be used. The reaction proceeds even without solvents but, when a solvent is to be used, aromatic hydrocarbons such as benzene, toluene and the like, ethers such as ethyl ether, tetrahydrofuran and the like, alkyl halides such as dichloromethane, chloroform and the like, water and the like can be used. The reaction temperature is preferably 0 °C to 100 °C.

And, the pyridine derivative represented by the general formula (I-12) in which $R^7$ is an alkyl group can be prepared by the reaction of an N-monosubstituted amide represented by the general formula

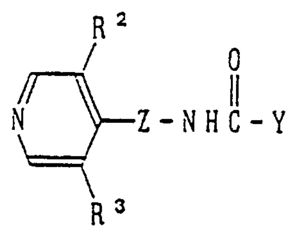

...... (XXXIII)

(in the formula, $R^2$, $R^3$, Y and Z are each the same as given above) and a halide compound represented by the general formula

$$R^7\text{-}X \qquad \cdots\cdots \quad (XXXIV)$$

(in the formula, $R^7$ and X are each the same as given above) in a solvent in the presence of a base to effect alkylation. The base used in the reaction is exemplified by metallic sodium, sodium amide, sodium hydride, potassium hydroxide and the like. The solvent to be used is exemplified by aromatic hydrocarbons such as benzene, toluene and the like, polar aprotic solvents such as dimethyl formamide, dimethyl sulfoxide and the like, and so on. The reaction temperature is preferably 0 °C to 100 °C.

The pyridine derivative represented by the general formula

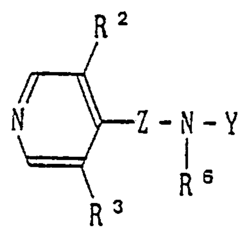

...... (I-13)

(in the formula, $R^2$, $R^3$, $R^6$, Y and Z are each the same as given above) can be prepared by the reaction of an amine represented by the general formula

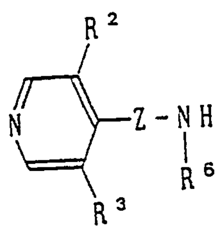

...... (XXXV)

(in the formula, $R^2$, $R^3$, $R^6$ and Z are each the same as given above with an alkylating agent represented by the general formula

$$Y\text{-}X'' \qquad \cdots\cdots \quad (XXI)$$

in a solvent in the presence of a base. The base used in the reaction includes sodium hydroxide, sodium amide, alkyl lithiums, aryl lithiums and the like. The solvent used in the reaction is exemplified by ethers, alcohols, water and the like. The reaction temperature is preferably 0 °C to 100 °C.

The pyridine derivative represented by the general formula

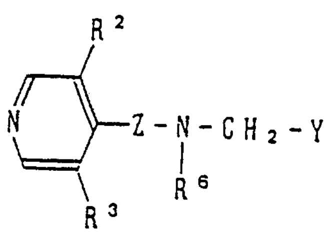

...... (I-13')

(in the formula, $R^2$, $R^3$, $R^6$, Y and Z are each the same as given above) can be prepared by the reduction of the carbonyl group in the amide compound represented by the general formula

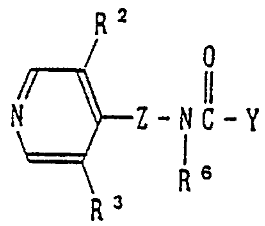

...... (XXXVI)

(in the formula, $R^2$, $R^3$, $R^6$, Y and Z are each the same as given above) with a reducing agent under a solvent. The reducing agent used in the reaction is exemplified by aluminum lithium hydride, sodium borohydride, diborane and the like. The solvent used in the reaction is exemplified by ethers, alcohols and the like. The reaction temperature is preferably 0 °C to 100 °C.

In the pyridine derivative represented by the above given general formula (I), incidentally, the object of extending the alkyl chain in $R^2$ and $R^3$ can be achieved by the reaction with an alkylating agent. This reaction can be performed by a known alkylating method. The alkylating agent used here is exemplified by methyl chloride, methyl bromide, methyl iodide, ethyl chloride, ethyl bromide, ethyl iodide, isopropyl chloride, isopropyl bromide, isopropyl iodide, sec-butyl chloride, sec-butyl bromide, sec-butyl iodide, n-amyl bromide and the like.

The pyridine derivative of the present invention can form a pyridinium salt with an acid so that the present invention also provides a salt of the pyridine derivative. The acid here is exemplified, for example, by hydrochloric acid, hydrobromic acid, hydroiodic acid, hydrofluoric acid, sulfuric acid, nitric acid, phosphoric acid, citric acid, lactic acid, oxalic acid, maleic acid, tartaric acid, benzoic acid, nicotinic acid, dodecyl benzene sulfonic acid, various kinds of fatty acids and the like.

The insecticidal-acaricidal agent of the present invention comprises the pyridine derivative represented by the above given general formula (I) or a salt thereof as the effective ingredient.

The insecticidal-acaricidal agent of the present invention is effective for the control of the pests on various kinds of agricultural crops and horticultural crops, hygienic pests and the like and the activity is exhibited to the insects of Hemiptera, Coloptera, Lepidoptera, Acarina and the like. Typical insects include Myzus persicae, Aphys gossypii, Lipaphis erysimi, Naphotettix cincticeps, Nilaparvata lugens, Sogatella furcifera, Laodelphax striatellus, Trialeurodas vaporariorum, Henosepilachna vigintioctopunctata, Oulema oryzae, Lissorhoptrus oryzophilus, Cnaphalocrosis medinalis, Tetranychus urticae, Panonychus citri and the like.

The insecticidal-acaricidal agent of the present invention is particularly effective for the control of the paddy field pests such as unka, yokobai and the like. Furthermore, it is also effective for the control of the pests on various kinds of dry field crops, trees, lawn grass, pasture grass, harvested grains, woods and wooden wares.

In the preparation of the formulated forms of the insecticidal-acaricidal agent of the present invention, various types of formulated forms can be obtained including solids, liquids, pastes and the like containing the effective ingredient and the actual forms include dusts, granules, beads, water-dispersible powders, oily preparations, emulsions, aerosols, flowables and the like.

The dusts can be prepared by blending the effective ingredient and a solid carrier and pulverizing the same. Granules or beads can be prepared, for example, by coating or impregnating a preliminarily shaped granular solid carrier with the effective ingredient or, alternatively, by the techniques of agglomeration to bind the effective ingredient to the solid carrier.

Here, the solid carrier includes powders of vegetable origin such as grains, soybeans, wood, bark, wheat bran and the like and mineral powders such as clay, talc, bentonite, acid clay, kaolin, diatomaceous earth, synthetic silicates, pumice stone, activated carbon, fly ash and the like as well as synthetic resins and the like.

The solid formulated form can be in the form of a dispersible or water-dispersible solid preparation such as a water-dispersible powder in which dispersion of the effective ingredient into liquids is promoted by mixing, besides the effective ingredient and the solid carrier, one kind or more of surface active agents working as a moisturizing agent, emulsifier and/or dispersion aid. Here, usable surface active agents include cationic, anionic and non-ionic surface active agents and the like. The cationic surface active agents are exemplified by quaternary ammonium salts such as cetyl trimethyl ammonium bromide and the like. And, the anionic surface acitve agents are exemplified by salts of alkyl aryl sulfonic acid, salts of lignin sulfonic acid and the like and the non-ionic surface active agents are exemplified by polyoxyethylene alkyl aryl ethers, polyoxyethylene higher fatty acid esters, sorbitan esters, sucrose esters and the like.

In the next place, the liquid formulated form consists of a solution or a dispersion of the effective ingredient in a liquid carrier which may optionally contain one kind or more of surface active agents working as a moisturizing agent, emulsifier and/or dispersion aid as mentioned above.

The liquid carrier is exemplified, besides water, by alcohols such as methanol, ethanol, ethylene glycol and the like, ketones such as methyl ethyl ketone, diisobutyl ketone, cyclohexanone and the like, hydrocarbons such as kerosene, solvent naphtha, toluene, xylene and the like, esters such as dioctyl phthalate and the like, amides such as dimethyl formamide and the like, nitriles such as acetonitrile and the like, dimethyl sulfoxide, fats and oils and the like.

The insecticidal-acaricidal agent of the present invention can further contain fixing agents, thickening agents, stabilizers and the like as an adjuvant and the adjuvants of this kind include casein, gelatin, arginic acid, carboxymethyl cellulose, gum arabic, polyvinyl alcohol and the like.

The insecticidal-acaricidal agent of the present invention can be either a ready-to-use preparation or a concentrated preparation to be diluted before use and can contain 0.1 to 99% by weight or, preferably, 0.5 to 80% by weight of the pyridine derivative or a salt thereof as the effective ingredient of the present invention. For example, it is appropriate that the dusts and granules contain from 0.5 to 20% by weight of the pyridine derivative or a salt thereof as the effective ingredient of the present invention and emulsions and water-dispersible powders contain from 5 to 50% by weight of the same.

In the following, examples of formulated preparations of various forms are shown below.

① Example of 50% emulsion

| Composition | Ratio of formulation (parts by weight) |
|---|---|
| Compound as the effective ingredient of the present invenion | 50 |
| Xylene | 40 |
| Mixture of polyoxyethylene nonylphenyl ether and calcium alkylbenzene sulfonate | 10 |

The above mentioned three ingredients are thoroughly agitated and mixed to give an emulsion.

② Example of 3% dust

| | |
|---|---|
| Compound as the effective ingredient of the present invention | 3 |
| Clay powder | 97 |

The above mentioned ingredients are thoroughly pulverized and mixed to give a dust preparation.

③ Example of 20% water-dispersible powder

| | |
|---|---|
| Compound as the effective ingredient of the present invention | 20 |
| Anionic surface active agent | 5 |
| Diatomaceous earth | 75 |

The above given ingredients are thoroughly pulverized and mixed to give a water-dispersible powder.

④ Example of 2% oily preparation

| | |
|---|---|
| Compound as the effective ingredient of the present invention | 2 |
| Kerosene | 98 |

The above given ingredients are thoroughly blended to give an oily preparation.

⑤ Example of 5% granules

| | |
|---|---|
| Compound as the effective ingredient of the present invention | 5 |
| Bentonite | 53 |
| Talc | 40 |
| Calcium lignin sulfonate | 2 |

The above given ingredients are thoroughly pulverized and mixed and then kneaded with addition of water followed by granulation and drying to give granules.

Though dependent on various factors such as the objective pests, circumstances of their development, weather, preparation form, method of application, site of application, season of application and the like, the standard dose of use of the insecticidal-acaricidal agent of the present invention is usually 1 to 10 kg of the preparation per 10 ares for dusts and granules. And, when used finally in the form of a liquid as is the case with emulsions and water-dispersible powders, the liquid for sprinkling is prepared usually by dilution such that the concentration of the effective ingredient is at least 0.001% by weight.

When the insecticidal-acaricidal agent of the present invention is further admixed with other known insecticides, acaricides, insect hormone agents, bactericides, nematicides, herbicides, plant-growth controlling agents, fertilizers and the like, a multi-purpose composition of further increased effects can be prepared and a synergistic effect can also be expected.

Particular examples of the insecticides suitable for admixture include pyrethroids such as permethorin, phenvalerate, esphenvalerate, cycloprothorin, biphenthorin, phenpropathrin, ethophenprox and the like, organic phosphorus compounds such as phenthion, phenthoate, diazine, MEP, DDVP, malathon, dimethoate, DMTP, acephate and the like, carbamate compounds such as NAC, MTMC, PHC, MPMC, BPMC, mesomil, carbosulfan, caltap, oxamil and the like and benzoyl urea compounds such as chlorofluazlon, theflubenzron and the like as well as phenbuta tin oxide, amitraz, chlorobenzylate, phenoxy carp,

buprophezin and the like.

Particular examples of the bactericides include kasugamycin, blastcydin S, fusaride, IBP, EDDP, tricyclazol, pyroxin, isoprothiolan, validamycin, polyoxin, mepronyl, flutranyl, pencyclon, dichloromezin, thiophanate methyl, prosimidon, iprodion, triazimephon, bitertanol, phenarimol, prochloraz, triflumizol, pyriphenox, metharaxyl, phosetyl, guazathin and the like.

[Examples]

In the following, the present invention is described in more detail by way of examples and comparative examples. Example 1.

Into a 200 ml flask were introduced 0.83 g (8.26 m moles) of diisopropyl amine and 15 ml of tetrahydrofuran and chilled at -50 °C. Under a stream of nitrogen, 5.5 ml (8.26 m moles) of n-butyl lithium (15% n-hexane solution) were added thereto and agitated for 10 minutes followed by dropwise addition of a tetrahydrofuran solution of 1.0 g (8.26 m moles) of 3-ethyl-4-methyl pyridine. After agitation for 30 minutes at -50 °C, the reaction temperature was gradually increased and, after keeping for 30 minutes at -10 °C, it was again chilled to -50 °C. A tetrahydrofuran solution of 1.25 g (8.26 m moles) of n-amyl bromide was added dropwise thereto and agitated for 30 minutes at -50 °C followed by return to room temperature. In the next place, water was added to distil off tetrahydrofuran under reduced pressure followed by extraction with ethyl acetate, washing with a saturated aqueous solution of sodium chloride and drying over anhydrous sodium sulfate. The solvent was distilled off under reduced pressure and purification by silica gel column chromatography was undertaken to give 1.03 g (yield 65%) of the desired 3-ethyl-4-n-hexyl pyridine. The structural formula of the thus obtained substance, yield and analytical results are shown in Tables 1 and 2.

Examples 2 to 29.

In Example 1, compounds were obtained by conducting the same procedure as in Example 1 excepting the use of the specified amounts of the pyridine compound and alkyl halide as shown in Table 1. The structural formulas of the thus obtained compounds, yields and analytical results are shown in Tables 1 and 2.

Table 1

| Example No. | Alkyl halide used | Amount of alkyl halide used (g) | Pyridine compound used | Amount of pyridine compound used (g) | Yield (g) | Yield (%) | Values of elementary analysis* (%) | | |
|---|---|---|---|---|---|---|---|---|---|
| | | | | | | | C | H | N |
| 1 | n-amyl bromide | 1.25 | 3-ethyl-4-methyl pyridine | 1.0 | 1.03 | 65 | 81.30 (81.62) | 11.65 (11.06) | 7.05 (7.32) |
| 2 | 2,4,4-trimethyl amyl bromide | 1.6 | 〃 | 〃 | 1.73 | 90 | 82.56 (82.34) | 11.56 (11.66) | 5.88 (6.00) |
| 3 | 1-bromoundecane | 1.9 | 〃 | 〃 | 1.32 | 58 | 82.47 (82.84) | 12.16 (12.07) | 5.37 (5.08) |
| 4 | 1-bromooctadecane | 2.75 | 〃 | 〃 | 1.57 | 51 | 83.81 (83.57) | 12.54 (12.68) | 3.65 (3.75) |
| 5 | ethyl iodide | 1.3 | 3,4-diethyl pyridine | 1.1 | 0.55 | 41 | 81.16 (80.93) | 10.22 (10.50) | 8.62 (8.58) |
| 6 | n-butyl bromide | 1.0 | 〃 | 〃 | 0.64 | 40 | 82.09 (81.62) | 10.54 (11.06) | 7.38 (7.32) |
| 7 | n-amyl bromide | 1.25 | 〃 | 〃 | 0.73 | 43 | 81.39 (81.89) | 11.86 (11.29) | 6.75 (6.82) |
| 8 | n-hexyl iodide | 1.36 | 〃 | 〃 | 0.52 | 29 | 82.45 (82.13) | 11.07 (11.49) | 6.48 (6.38) |
| 9 | n-nonyl bromide | 1.7 | 〃 | 〃 | 0.52 | 24 | 82.68 (82.69) | 12.21 (11.95) | 5.10 (5.36) |
| 10 | ethyl iodide | 1.3 | 3-methyl-4-(1,3,5,5-tetra-methylhexyl)-pyridine | 1.92 | 0.43 | 20 | 82.98 (82.69) | 11.60 (11.95) | 5.41 (5.36) |

Table 1 (continued)

| Example No. | Alkyl halide used | Amount of alkyl halide used (g) | Pyridine compound used | Amount of pyridine compound used (g) | Yield (g) | Yield (%) | Value of elementary analysis* (%) | | |
|---|---|---|---|---|---|---|---|---|---|
| | | | | | | | C | H | N |
| 11 | n-propyl bromide | 1.0 | 3-methyl-4-(1,3,5,5-tetramethylhexyl)-pyridine | 1.92 | 1.73 | 76 | 89.96 (82.84) | 12.04 (12.07) | 4.99 (5.08) |
| 12 | isopropyl iodide | 1.4 | 〃 | 〃 | 0.80 | 35 | 83.10 (82.84) | 11.94 (12.07) | 4.96 (5.08) |
| 13 | n-amyl bromide | 1.25 | 〃 | 〃 | 2.05 | 82 | 82.92 (83.10) | 12.49 (12.29) | 4.59 (4.61) |
| 14 | 4,4-dimethylamyl bromide | 1.5 | 3-ethyl-4-methyl pyridine | 1.0 | 0.47 | 26 | 82.41 (82.13) | 11.16 (11.49) | 6.43 (6.38) |
| 15 | 3,3-dimethylamyl bromide | 1.5 | 〃 | 〃 | 0.69 | 38 | 82.45 (82.13) | 11.02 (11.49) | 6.53 (6.38) |
| 16 | 4-methylamyl bromide | 1.36 | 〃 | 〃 | 1.52 | 90 | 82.05 (81.89) | 11.24 (11.29) | 6.71 (6.82) |
| 17 | 1-bromo-3,3-dimethyl hexane | 1.6 | 〃 | 〃 | 0.89 | 57 | 82.63 (82.34) | 11.46 (11.66) | 5.91 (6.00) |
| 18 | 2-ethylhexyl bromide | 1.6 | 〃 | 〃 | 1.17 | 61 | 82.45 (82.34) | 11.62 (11.66) | 5.93 (6.00) |
| 19 | 1-bromo-3,5,5-trimethyl hexane | 1.7 | 〃 | 〃 | 1.33 | 65 | 82.35 (82.53) | 12.07 (11.81) | 5.57 (5.66) |
| 20 | 〃 | 〃 | 3,4-diethyl pyridine | 1.1 | 0.50 | 23 | 82.49 (82.69) | 12.31 (11.95) | 5.20 (5.36) |

Table 1 (continued)

| Example No. | Alkyl halide used | Amount of alkyl halide used (g) | Pyridine compound used | Amount of pyridine compound used (g) | Yield (g) | Yield (%) | Values of elementary analysis* (%) C | H | N |
|---|---|---|---|---|---|---|---|---|---|
| 21 | 1-bromo-4,6-dimethyl heptane | 1.7 | 3-ethyl-4-methyl pyridine | 1.0 | 1.06 | 52 | 82.40 (82.53) | 11.84 (11.81) | 5.76 (5.66) |
| 22 | 1-bromo-4,6,8-trimethyl nonane | 2.06 | 〃 | 〃 | 1.41 | 59 | 82.63 (82.98) | 12.46 (12.19) | 4.92 (4.84) |
| 23 | 1-bromo-2-cyclopentyl-ethane | 1.46 | 〃 | 〃 | 1.54 | 86 | 82.59 (82.89) | 10.90 (10.67) | 6.51 (6.44) |
| 24 | 1-bromo-2-cyclohexyl ethane | 1.6 | 〃 | 〃 | 1.62 | 85 | 83.31 (83.06) | 10.70 (10.89) | 5.98 (6.05) |
| 25 | 1-bromo-3-cyclohexyl propane | 1.7 | 〃 | 〃 | 1.15 | 57 | 83.16 (83.20) | 11.24 (11.09) | 5.60 (5.71) |
| 26 | geranyl chloride | 1.4 | 〃 | 〃 | 0.74 | 35 | 83.84 (83.99) | 10.87 (10.57) | 5.29 (5.44) |
| 27 | citronelly bromide | 1.8 | 〃 | 〃 | 0.36 | 17 | 82.94 (83.33) | 11.50 (11.27) | 5.56 (5.40) |
| 28 | 1-bromo-2-isobutyloxy ethane | 1.36 | 〃 | 〃 | 0.69 | 38 | 75.69 (75.97) | 10.20 (10.47) | 6.52 (6.33) |
| 29 | 1-bromo-3-methoxy-3-methyl butane | 1.5 | 〃 | 〃 | 0.26 | 14 | 75.80 (75.97) | 10.19 (10.47) | 6.31 (6.33) |

* Calculated value shown by the numerical figure in brackets

Table 2

| Example | Structural formula | Chemical formula | Name of compound | Infrared absorption spectrum*1 ($cm^{-1}$) | Proton nuclear magnetic resonance spectrum*2 (ppm) |
|---|---|---|---|---|---|
| 1 | 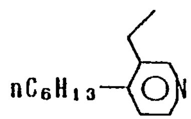 | $C_{13}H_{21}N$ | 3-ethyl-4-n-hexyl pyridine | 2890~3000 1606,1501 | 0.89(3H,t),1.23(3H,t),1.05~1.8(8H,m),2.61(2H,t),2.65(2H,q),7.03(1H,d),8.32(1H,d),8.35(1H,s) |
| 2 | 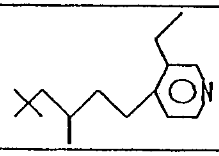 | $C_{16}H_{27}N$ | 3-ethyl-4-(3,5,5-trimethyl hexyl)-pyridine | 2890~3000 1600,1474 | 0.90(9H,s),1.01(3H,d),1.24(3H,t),1.1 ~1.7(5H,m),2.45~2.8(4H,m),7.02(1H,d),8.31(1H,d),8.34(1H,s) |
| 3 | 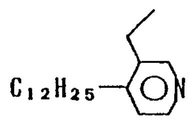 | $C_{19}H_{33}N$ | 3-ethyl-4-n-dodecanyl pyridine | 2850~3000 1590,1484 | 0.88(3H,t),1.0~1.7(23H,m),2.61(2H,t),2.65(2H,q),7.03(1H,d),8.32(1H,d),8.35(1H,s) |
| 4 | 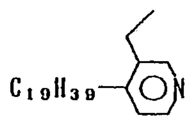 | $C_{26}H_{47}N$ | 3-ethyl-4-n-nonadecanyl pyridine | 2880~3000 1610,1486 | 0.88(3H,t),1.0~1.6(37H,m),2.61(2H,t),2.66(2H,q),7.03(1H,d),8.32(1H,d),8.36(1H,s) |
| 5 | 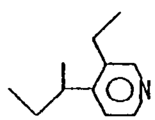 | $C_{11}H_{17}N$ | 3-ethyl-4-sec-butyl pyridine | 2900~3090 1604,1500 | 0.84(3H,t),1.21(3H,d),1.23(3H,t),1.4~1.8(2H,m),2.69(2H,q),2.90(1H,q),7.08(1H,d),8.36(1H,s),8.38(1H,d) |
| 6 | 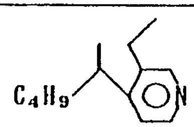 | $C_{13}H_{21}N$ | 2-(3-ethyl-4-pyridyl)-hexane | 2880~3000 1600,1495 | 0.85(3H,t),1.21(3H,d),1.22(3H,t),1.1~1.8(6H,m),2.69(2H,q),2.97(1H,q),7.09(1H,d),8.35(1H,s),8.38(1H,d) |

Table 2 (continued)

| Example | Structural formula | Chemical formula | Name of compound | Infrared absorption spectrum*1 ($cm^{-1}$) | Proton nuclear magnetic resonance spectrum*2 (ppm) |
|---|---|---|---|---|---|
| 7 | 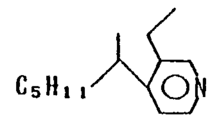 | $C_{14}H_{23}N$ | 2-(3-ethyl-4-pyridyl)-heptane | 2890～3000<br>1604,1500 | 0.85(3H,t),1.0～1.75(14H,m),2.67(2H,q),2.96(1H,q),7.08(1H,d),8.35(1H,s),8.37(1H,d) |
| 8 | 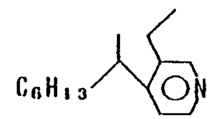 | $C_{15}H_{25}N$ | 2-(3-ethyl-4-pyridyl)-octane | 2880～3000<br>1601,1498 | 0.86(3H,t),1.0～1.8(16H,m),2.67(2H,q),2.96(1H,q),7.08(1H,d),8.35(1H,s),8.37(1H,d) |
| 9 | 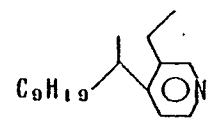 | $C_{18}H_{31}N$ | 2-(3-ethyl-4-pyridyl)-undecane | 2860～3000<br>1598,1492 | 0.87(3H,t),1.0～1.75(22H,m),2.67(2H,q),2.96(1H,q),7.08(1H,d),8.35(1H,s),8.37(1H,d) |
| 10 | 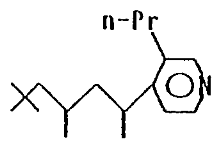 | $C_{18}H_{31}N$ | 2-(3-n-propyl-4-pyridyl)-4,6,6-trimethyl heptane | 2900～3000<br>1603,1480 | 0.82(9H,s),0.88(3H,t),0.9 ～1.8(13H,m),2.4 ～2.75(2H,m),3.04(1H,q),7.09(1H,d),8.33(1H,d),8.35(1H,d) |
| 11 | 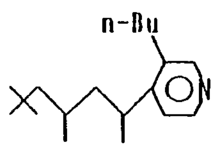 | $C_{19}H_{33}N$ | 2-(3-n-butyl-4-pyridyl)-4,6,6-trimethyl heptane | 2870～3000<br>1598,1465 | 0.83(9H,s),0.88(3H,t),0.9 ～1.8(15H,m),2.64(2H,t),3.03(1H,q),7.09(1H,d),8.32(1H,s),8.35(1H,d) |
| 12 | 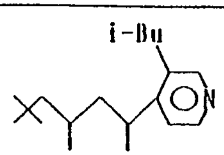 | $C_{17}H_{33}N$ | 2-(3-iso-butyl-4-pyridyl)-4,6,6-trimethyl heptane | 2890～3000<br>1603,1480 | 0.83(9H,s),0.86(6H,d),0.9 ～2.0(12H,m),2.52(2H,t),3.02(1H,q),7.10(1H,d),8.29(1H,s),8.36(1H,d) |

Table 2 (continued)

| Example | Structural formula | Chemical formula | Name of compound | Infrared absorption spectrum*1 ($cm^{-1}$) | Proton nuclear magnetic resonance spectrum*2 (ppm) |
|---|---|---|---|---|---|
| 13 | 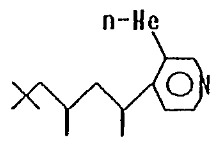 | $C_{21}H_{37}N$ | 2-(3-n-hexyl-4-pyridyl)-4,6,6-trimethyl heptane | 2890~3000 1600,1486 | 0.83 (9H, s), 0.88 (3H, t), 0.9 ~1.7 (19H, m), 2.65 (2H, t), 3.03 (1H, q), 7.09 (1H, d), 8.32 (1H, s), 8.35 (1H, d) |
| 14 | 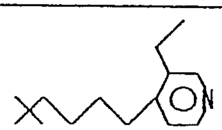 | $C_{15}H_{25}N$ | 3-ethyl-4-(5,5-dimethylhexyl)-pyridine | 2890~3000 1606,1480 | 0.88 (9H, s), 1.23 (3H, t), 1.1 ~1.7 (6H, m), 2.66 (4H, q), 7.03 (1H, d), 8.32 (1H, d), 8.35 (1H, s) |
| 15 | 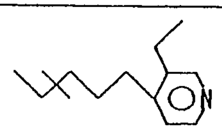 | $C_{15}H_{25}N$ | 3-ethyl-4-(4,4-dimethylhexyl)-pyridine | 2890~3060 1600,1495 | 0.79 (3H, t), 0.82 (6H, s), 1.22 (3H, t), 1.0 ~1.8 (6H, m), 2.45~2.8 (4H, m), 7.04 (1H, d), 8.22 (1H, d), 8.35 (1H, s) |
| 16 | 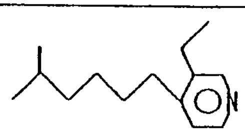 | $C_{14}H_{23}N$ | 3-ethyl-4-(5-methylhexyl)-pyridine | 2890~3000 1602,1500 | 0.88 (6H, d), 1.23 (3H, t), 1.1 ~2.8 (6H, m), 2.65 (4H, q), 7.03 (1H, d), 8.23 (1H, d), 8.35 (1H, s) |
| 17 | 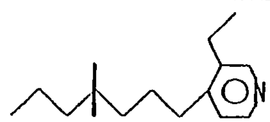 | $C_{16}H_{27}N$ | 3-ethyl-4-(4,4-dimethylheptyl)-pyridine | 2900~3050 1608,1502 | 0.83 (6H, s), 0.87 (3H, t), 1.23 (3H, t), 1.1 ~1.75 (8H, m), 2.52 (2H, q), 2.65 (2H, q), 7.05 (1H, d), 8.33 (1H, d), 8.36 (1H, s) |
| 18 | 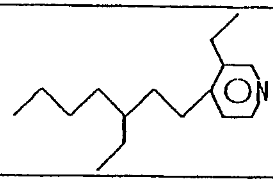 | $C_{16}H_{27}N$ | 3-ethyl-4-(3-ethylheptyl)-pyridine | 2880~3000 1600,1485 | 0.75~1.1 (6H, m), 1.24 (3H, t), 1.1~1.7 (13H, m), 2.45~2.8 (4H, m), 7.04 (1H, d), 8.32 (1H, d), 8.36 (1H, s) |

Table 2 (continued)

| Example | Structural formula | Chemical formula | Name of compound | Infrared absorption spectrum*1 ($cm^{-1}$) | Proton nuclear magnetic resonance spectrum*2 (ppm) |
|---|---|---|---|---|---|
| 19 | 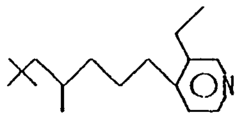 | $C_{17}H_{29}N$ | 3-ethyl-4-(4,6,6-trimethylheptyl)-pyridine | 2880～3000 1585,1467 | 0.89(9H,s),0.92(3H,d),1.23(3H,t), 1.0 ～1.8(7H,m),2.45～2.8(4H,m), 7.04(1H,d),8.33(1H,d),8.35(1H,s) |
| 20 | 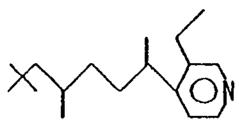 | $C_{18}H_{31}N$ | 2-(3-ethyl-4-pyridyl)-5,7,7-trimethyl octane | 2870～3000 1590,1464 | 0.85(9H,m),0.88(3H,d),1.0 ～1.8 (13H,m),2.5 ～3.1(3H,m),7.08(1H,d), 8.35(1H,s),8.37(1H,d) |
| 21 | 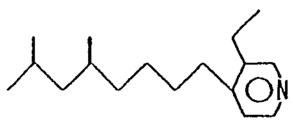 | $C_{17}H_{29}N$ | 3-ethyl-4-(5,7-dimethyloctyl)-pyridine | 2890～3000 1601,1497 | 0.84(6H,d),0.87(3H,d),1.23(3H,t),1.0 ～1.8(10H,m),2.62(2H,t),2.66(2H,q), 7.04(1H,d),8.32(1H,d),8.35(1H,s) |
| 22 | 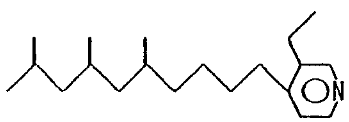 | $C_{20}H_{35}N$ | 3-ethyl-4-(5,7,9-trimethyldecanyl) pyridine | 2880～3000 1600,1495 | 0.7 ～1.8(28H,m),2.62(2H,t), 2.66(2H,q),7.04(1H,d),8.32(1H,d), 8.35(1H,s) |
| 23 | 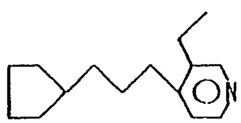 | $C_{15}H_{23}N$ | 3-ethyl-4-(3-cyclopentyl-propyl)-pyridine | 2900～3100 1610,1504 | 1.23(3H,t),0.8～1.9(13H,m),2.61 (2H,t),2.65(2H,q),7.03(1H,d),8.33 (1H,d),8.35(1H,s) |
| 24 | 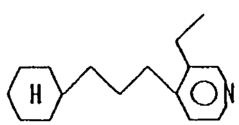 | $C_{16}H_{25}N$ | 3-ethyl-4-(3-cyclohexylpropyl)-pyridine | 2860～3000 1606,1495 | 0.7 ～1.9(15H,m),1.22(3H,t),2.45～ 2.8(4H,m),7.03(1H,d),8.32(1H,d), 8.35(1H,s) |

Table 2 (continued)

| Example | Structural formula | Chemical formula | Name of compound | Infrared absorption spectrum*1 ($cm^{-1}$) | Proton nuclear magnetic resonance spectrum*2 (ppm) |
|---|---|---|---|---|---|
| 25 | 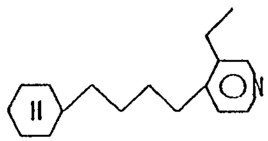 | $C_{17}H_{27}N$ | 3-ethyl-4-(4-cyclohexylbutyl)-pyridine | 2860～3000 1600,1497 | 0.7 ～1.8(17H,m),1.23(3H,t),2.61(3H,t),2.67(2H,q),7.02(1H,d), 8.32(1H,d),8.36(1H,s) |
| 26 | 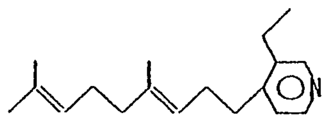 | $C_{18}H_{27}N$ | (E)-3-ethyl-4-(4,8-dimethyl-3,7-nonadienyl)-pyridine | 2900～3060 1602,1498 | 1.21(3H,t),1.5～1.8(9H,m),1.9 ～2.9(10H,m),5.09(2H,br t),6.97(1H,d), 8.20(1H,d),8.25(1H,s) |
| 27 | 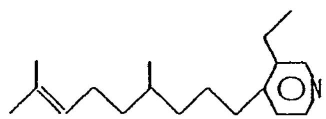 | $C_{18}H_{29}N$ | 3-ethyl-4-(4,8-dimethyl-7-nonaenyl)-pyridine | 2890～3000 1604,1500 | 0.88(3H,d),1.23(3H,t),1.60(3H,s), 1.69(3H,s),1.0～2.8(13H,m),5.10(1H,br t),7.04(1H,d),8.32(1H,d), 8.35(1H,s) |
| 28 | 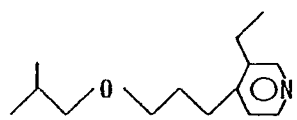 | $C_{14}H_{23}NO$ | 3-ethyl-4-(3-isobutyloxy-propyl)-pyridine | 2820～3000 1606,1497 | 0.92(6H,d),1.23(3H,t),1.6 ～2.1(3H,m),2.68(2H,q),2.72(2H,t),3.18(2H,d),3.44(2H,t),7.06(1H,d),8.33(1H,d),8.36(1H,s) |
| 29 | 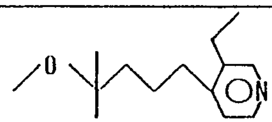 | $C_{14}H_{23}NO$ | 3-ethyl-4-(4-methoxy-4-methyl-pentyl)-pyridine | 2840～3000 1600,1496 | 1.14(6H,s),1.23(3H,t),1.5 ～1.7(4H,m),2.66(4H,q),3.15(3H,s),7.06(1H,d),8.33(1H,d),8.36(1H,s) |

*1 by the potassium bromide disc method
*2 intermal standard: tetramethyl silane

Examples 30 to 108.

In Example 1, compounds were obtained by conducting the same procedure as in Example 1 excepting the use of the pyridine compound and the alkyl halide shown in Tables 3 and 4. The structural formulas of the thus obtained compounds, yields and others are shown in Tables 3 and 4.

Table 3

| Example | Alkyl halide used | Pyridine compound used | Yield (%) | Structural formula | Name of compound |
|---|---|---|---|---|---|
| 30 | 2-isobutyl-4-methyl pentyl bromide | 3-ethyl-4-methyl pyridine | 84.8 | 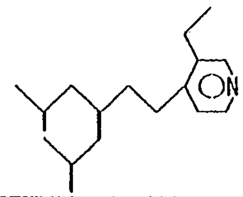 | 1-(3-ethyl-4-pyridyl)-3-isobutyl-4-methyl hexane |
| 31 | 2-n-butyl-hexyl bromide | 〃 | 81.6 | 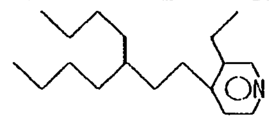 | 1-(3-ethyl-4-pyridyl)-3-n-butyl heptane |
| 32 | 4,4-dimethylpentyl bromide | 3,4-diethyl pyridine | 20.8 | 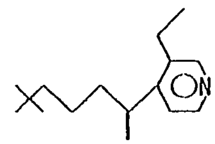 | 2-(3-ethyl-4-pyridyl)-6,6-dimethyl heptane |
| 33 | 4-ethyl-hexyl bromide | 3-ethyl-4-methyl pyridine | 82.6 | 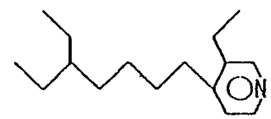 | 1-(3-ethyl-4-pyridyl)-4-ethyl heptane |
| 34 | tetradecanyl bromide | 〃 | 83.5 | 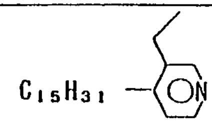 | 1-(3-ethyl-4-pyridyl)-pentadecane |
| 35 | 4,4-dimethylpentyl bromide | 5,6,7,8-tetra-hydro isoquinoline | 6.1 | 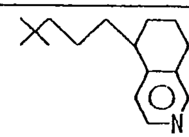 | 5-(4,4-dimethylpentyl)-5,6,7,8-tetrahydro isoquinoline |

Table 3 (continued)

| Example | Alkyl halide used | Pyridine compound used | Yield (%) | Structural formula | Name of compound |
|---|---|---|---|---|---|
| 36 | n-pentyl bromide | 5,6,7,8-tetra-hydro isoquinoline | 21.9 | 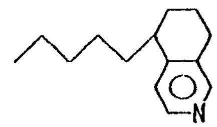 | 5-n-pentyl-5,6,7,8-tetrahydro isoquinoline |
| 37 | 3-pentynyl bromide | 3-ethyl-4-methyl pyridine | 11.5 | 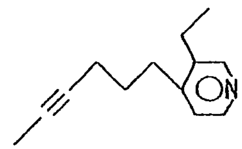 | 1-(3-ethyl-4-pyridyl)-4-hexyne |
| 38 | 2-pentyl bromide | 〃 | 24.2 | 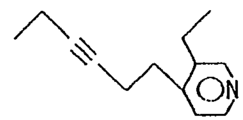 | 1-(3-ethyl-4-pyridyl)-3-hexyne |
| 39 | 3-butenyl bromide | 〃 | 98.0 | 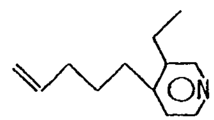 | 1-(3-ethyl-4-pyridyl)-4-pentene |
| 40 | 3,4-dimethylpentyl bromide | 〃 | 8.0 | 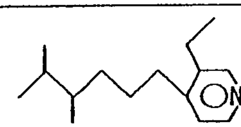 | 1-(3-ethyl-4-pyridyl)-4,5-dimethyl hexane |
| 41 | 3,4-dimethylhexyl bromide | 〃 | 51.9 | 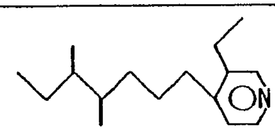 | 1-(3-ethyl-4-pyridyl)-4,5-dimethyl heptane |

Table 3 (continued)

| Example | Alkyl halide used | Pyridine compound used | Yield (%) | Structural formula | Name of compound |
|---|---|---|---|---|---|
| 42 | 5,7,7-trimethyl-octenyl bromide | 3-ethyl-4-methyl pyridine | 71.3 | 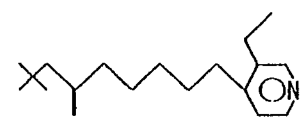 | 1-(3-ethyl-4-pyridyl)-6,8,8-trimethyl nonane |
| 43 | 6-methylheptyl bromide | 〃 | 50.1 | 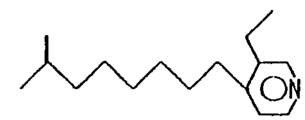 | 1-(3-ethyl-4-pyridyl)-7-methyl octane |
| 44 | 3,5,5-trimethylhexyl bromide | 3,4-dimethyl-5-ethyl pyridine | 10.4 | 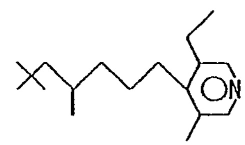 | 1-(3-ethyl-4-methyl-4-pyridil)-4,6,6-trimethyl heptane |
| 45 | 2-cyclohexylethyl bromide | 3,4-diethyl pyridine | 44.6 | 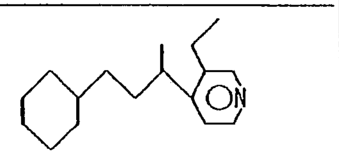 | 2-(3-ethyl-4-pyridyl)-4-cyclohexyl butane |
| 46 | 2-(2-norbornyl)-ethyl bromide | 3-ethyl-4-methyl pyridine | 51.0 | 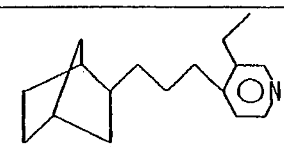 | 1-(3-ethyl-4-pyridyl)-3-(2-norbonyl)-propane |
| 47 | 2-(1-adamantyl)-ethyl bromide | 〃 | 85.2 | 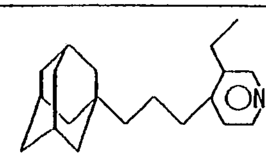 | 1-(3-ethyl-4-pyridyl)-3-(1-adamantyl)-propane |

Table 3 (continued)

| Example | Alkyl halide used: | Pyridine compound used | Yield (%) | Structural formula | Name of compound |
|---|---|---|---|---|---|
| 48 | 2-(2-t-butylcyclo-hexyl)-ethyl bromide | 3-ethyl-4-methyl pyridine | 53.4 | 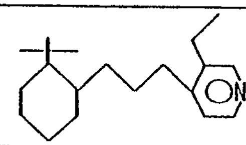 | 1-(3-ethyl-4-pyridyl)-3-(2-t-butylcyclohexyl)-propane |
| 49 | 2-(3,3,5-trimethyl-cyclohexyl)-ethyl bromide | 〃 | 52.6 | 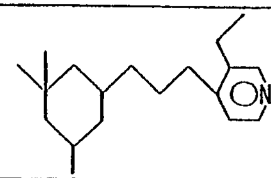 | 1-(3-ethyl-4-pyridyl)-3-(3,3,5-trimethylcyclo-hexyl)-propane |
| 50 | 2-(4-n-butylcyclo-hexyl)-ethyl bromide | 〃 | 84.1 | 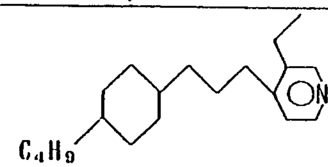 | 1-(3-ethyl-4-pyridyl)-3-(4-n-butylcyclohexyl)-propane |
| 51 | 2-(4-n-pentyl-1-cyclohexnyl)-ethyl bromide | 〃 | 26.6 | 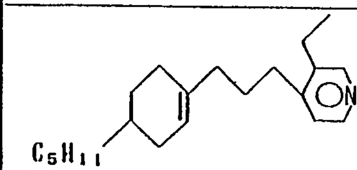 | 1-(3-ethyl-4-pyridyl)-3-4-n-pentyl-1-cyclohexnyl)-propane |
| 52 | 2-cycloheptyl ethyl bromide | 〃 | 16.8 | 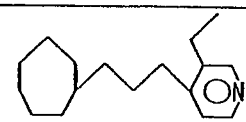 | 1-(3-ethyl-4-prydiyl)-3-cycloheptyl propane |
| 53 | 3-(4-t-butylcyclo-hexyl)-propyl bromide | 〃 | 59.0 | 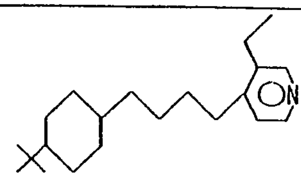 | 1-(3-ethyl-4-pyridyl)-4-(4-t-butylcyclo-hexyl)-butane |

Table 3 (continued)

| Example | Alkyl halide used | Pyridine compound used | Yield (%) | Structural formula | Name of compound |
|---|---|---|---|---|---|
| 54 | 3-cyclohexylbutyl bromide | 3-ethyl-4-methyl pyridine | 17.4 | 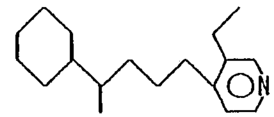 | 1-(3-ethyl-4-pyridyl)-4-cyclohexyl pentane |
| 55 | 3-cyclohexyl-2-propenyl bromide | 〃 | 31.0 | 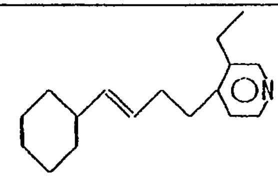 | (E)-1-(3-ethyl-4-pyridyl)-4-cyclohexyl-3-butene |
| 56 | 2-cyclopentylpropyl bromide | 〃 | 6.6 | 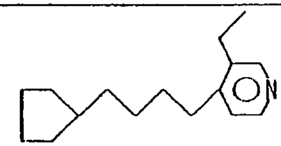 | 1-(3-ethyl-4-pyridyl)-3-cyclopentyl butane |
| 57 | 2-cyclooctylethyl bromide | 〃 | 22.2 | 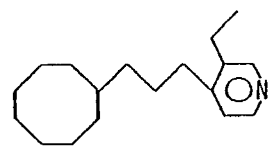 | 1-(3-ethyl-4-pyridyl)-3-cyclooctyl propane |
| 58 | 3-cyclobutylpropyl bromide | 〃 | 64.0 | 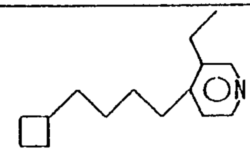 | 1-(3-ethyl-4-pyridyl)-4-cyclobutyl butane |
| 59 | 2-{2-(1,2,3,4-tetrahydronaphtyl)}-ethyl bromide | 〃 | 100.0 | 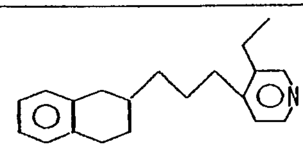 | 1-(3-ethyl-4-pyridyl)-3-{2-(1,2,3,4-tetarhydronaphthyl)}-propane |

Table 3 (continued)

| Example | Alkyl halide used | Pyridine compound used | Yield (%) | Structural formula | Name of compound |
|---|---|---|---|---|---|
| 60 | 3,6-dimethylheptyl bromide | 3-ethyl-4-methyl pyridine | 56.1 | 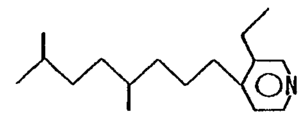 | 1-(3-ethyl-4-pyridyl)-4,7-dimethyl octane |
| 61 | 3,4-dimethylheptyl bromide | 〃 | 76.0 | 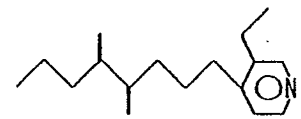 | 1-(3-ethyl-4-pyridyl)-4,5-dimethyl octane |
| 62 | 3,3-diethylpentyl bromide | 〃 | 14.6 | 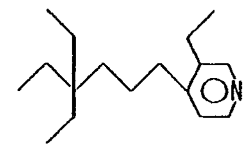 | 1-(3-ethyl-4-pyridyl)-4,4-diethyl hexane |
| 63 | 3,4,4-trimethylpentyl bromide | 〃 | 51.0 | 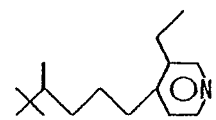 | 1-(3-ethyl-4-pyridyl)-4,5,5-trimethyl hexane |
| 64 | 5,5-dimethyl-3-hexenyl bromide | 〃 | 13.0 | 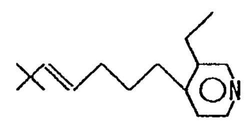 | (E)-1-(3-ethyl-4-pyridyl)-6,6-dimethyl-4-heptene |
| 65 | 4,5-dimethylheptyl bromide | 〃 | 21.2 | 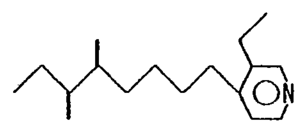 | 1-(3-ethyl-4-pyridyl)-5,6-dimethyl octane |

Table 3 (continued)

| Example | Alkyl halide used | Pyridine compound used | Yield (%) | Structural formula | Name of compound |
|---|---|---|---|---|---|
| 66 | 3,5-dimethylheptyl bromide | 3-ethyl-4-methyl pyridine | 32.1 | 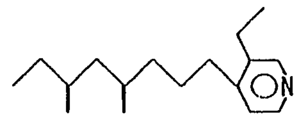 | 1-(3-ethyl-4-pyridyl)-4,6-dimethyl octane |
| 67 | 3-ethyl-5-methyl-heptyl bromide | 〃 | 31.5 | 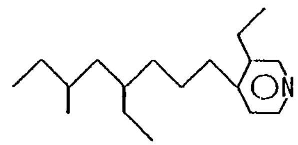 | 1-(3-ethyl-4-pyridyl)-4-ethyl-6-methyl octane |
| 68 | 3,5,5-trimethyl-hexyl bromide | 4-methyl-3-isopropyl pyridine | 28.5 | 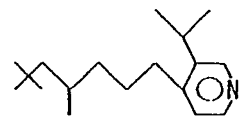 | 1-(3-isopropyl-4-pyridyl)-4,6,6-trimethyl heptane |
| 69 | 〃 | 3,5-diethyl-4-methylpyridine | 21.8 | 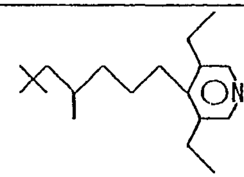 | 1-(3,5-diethyl-4-pyridyl)-4,6,6-trimethyl heptane |
| 70 | 4-ethylhexyl bromide | 3,4-diethyl-pyridine | 12.7 | 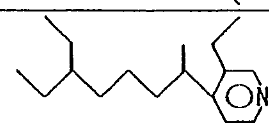 | 2-(3-ethyl-4-pyridyl)-6-ethyl octane |
| 71 | 3,5,5-trimethyl-hexyl bromide | 3-n-butyl-5-ethyl-4-methyl pyridine | 9.0 | 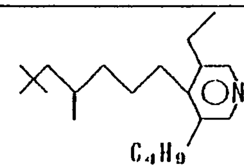 | 1-(3-n-butyl-5-ethyl-4-pyridyl)-4,6,6-trimethyl heptane |

Table 3 (continued)

| Example | Alkyl halide used | Pyridine compound used | Yield (%) | Structual formula | Name of compound |
|---|---|---|---|---|---|
| 72 | 3-ethylhexyl bromide | 3-ethyl-4-methyl pyridine | 78.2 | 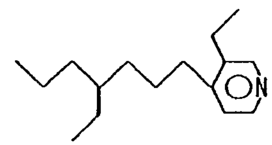 | 1-(3-ethyl-4-pyridyl)-4-ethyl heptane |
| 73 | 4-ethyl-2-methyl-hexyl bromide | 〃 | 55.2 | 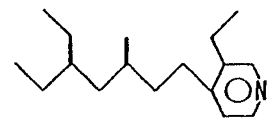 | 1-(3-ethyl-4-pyridyl)-5-ethyl-3-methyl heptane |
| 74 | 4,6,6-trimethyl-heptyl bromide | 〃 | 56.7 | 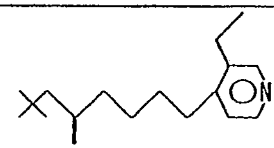 | 1-(3-ethyl-4-pyridyl)-5,7,7-trimethyl octane |
| 75 | 3,5,5-trimethyl-hexyl bromide | 3-ethyl-4-methyl-5-iso-propyl pyridine | 23.0 | 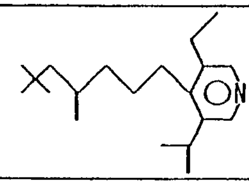 | 1-(3-ethyl-5-isopropyl-4-pyridyl)-4,6,6-tri-methyl heptane |
| 76 | 3-ethyl-2-pentenyl bromide | 3-ethyl-4-methyl pyridine | 53.7 | 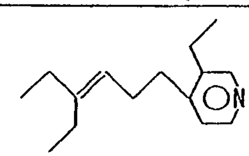 | 1-(3-ethyl-4-pyridyl)-4-ethyl-3-hexene |
| 77 | 3-cyclohexyl-butyl bromide | 3,4-dimethyl-5-ethyl pyridine | 49.5 | 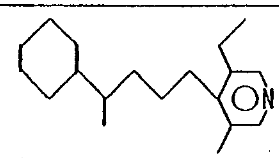 | 1-(3-ethyl-5-methyl-4-pyridyl)-4-cyclohexyl pentane |

Table 3 (contined)

| Example | Alkyl halide used | Pyridine compound used | Yield (%) | Structural formula | Name of compound |
|---|---|---|---|---|---|
| 78 | 2-(2-norbonyl)-ethyl bromide | 3,4-dimethyl-5-ethyl pyridine | 24.4 | 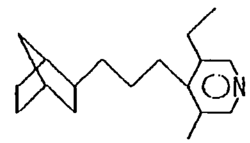 | 1-(3-ethyl-5-methyl-4-pyridyl)-3-(2-norbonyl)-propane |
| 79 | 2-cyclodecanyl-ethyl bromide | 3-ethyl-4-methyl pyridine | 85.2 | 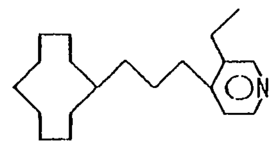 | 1-(3-ethyl-4-pyridyl)-3-cyclodecanyl propane |
| 80 | 2-cyclododecanyl-ethyl bromide | 〃 | 87.2 | 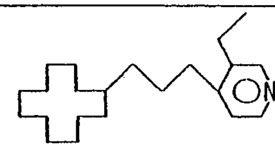 | 1-(3-ethyl-4-pyridyl)-3-cyclododecanyl propane |
| 81 | 2-bornylethyl bromide | 〃 | 84.5 | 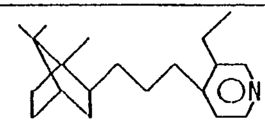 | 1-(3-ethyl-4-pyridyl)-3-bornyl propane |
| 82 | 2-(2-decahydro-naphthalenyl)-ethyl bromide | 〃 | 61.1 | 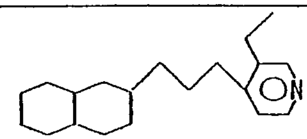 | 1-(3-ethyl-4-pyridyl)-3-(2-decahydronaphthalenyl)-propane |
| 83 | 2-{9-(bicyclo-[3,3,1]-nonyl)}-ethyl bromide | 〃 | 88.9 | 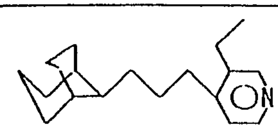 | 1-(3-ethyl-4-pyridyl)-3-[9-{bicyclo[3,3,1]-nonyl}-propane |

Table 3 (continued)

| Example | Alkyl halide used | Pyridine compound used | Yield (%) | Structural formula | Name of compound |
|---|---|---|---|---|---|
| 84 | 3-(3,3,5-trimethyl-cyclohexyl)-propyl bromide | 3-ethyl-4-methyl pyridine | 28.5 | 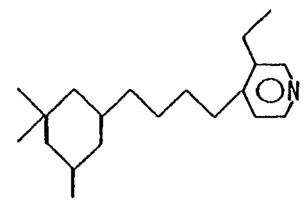 | 1-(3-ethyl-4-pyridyl)-4-(3,3,5-trimethylcyclo-hexyl)-butane |
| 85 | 3-cycloheptyl-propyl bromide | 〃 | 93.7 | 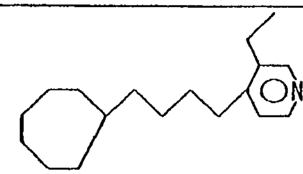 | 1-(3-ethyl-4-pyridyl)-4-cycloheptyl butane |
| 86 | 4-cycloheptylbutyl bromide | 〃 | 90.4 | 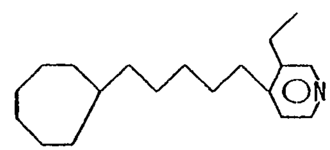 | 1-(3-ethyl-4-pyridyl)-5-cycloheptyl pentane |
| 87 | 5-methyl-4-hexenyl bromide | 〃 | 27.0 | 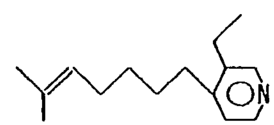 | 1-(3-ethyl-4-pyridyl)-6-methyl-5-heptene |
| 88 | 2-n-hexyloxyethyl bromide | 〃 | 34.0 | 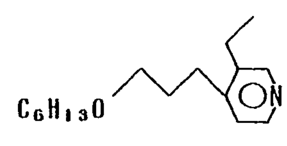 | 1-(3-ethyl-4-pyridyl)-3-n-hexyloxy propane |

Table 3 (continued)

| Example | Alkyl halide used | Pyridine compound used | Yield (%) | Structural formula | Name of compound |
|---|---|---|---|---|---|
| 89 | 2-isobutylthio-ethyl bromide | 3-ethyl-4-methyl pyridine | 47.7 | 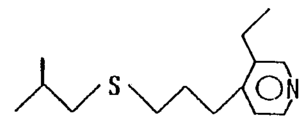 | 1-(3-ethyl-4-pyridyl)-3-isobutylthio propane |
| 90 | 〃 | 3,4-diethyl pyridine | 11.9 | 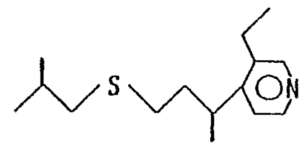 | 2-(3-ethyl-4-pyridyl)-4-isobutylthio butane |
| 91 | 2-[2-(1,3-dioxanyl)]-ethyl bromide | 3-ethyl-4-methyl pyridine | 83.9 | 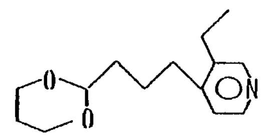 | 1-(3-ethyl-4-pyridyl)-3-[2-(1,3-dioxanyl)]-propane |
| 92 | 2-[2-methyl-2-(1,3-dithianyl)]-ethyl bromide | 〃 | 94.7 | 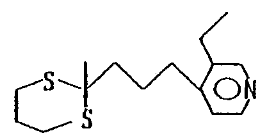 | 1-(3-ethyl-4-pyridyl)-3-[2-methyl-2-(1,3-dithianyl)]-propane |
| 93 | 2-tert-butyloxy-ethyl bromide | 〃 | 16.9 | 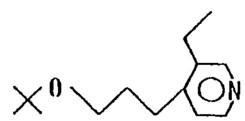 | 1-(3-ethyl-4-pyridyl)-3-t-butyloxy propane |
| 94 | 2-cyclohexyloxy-ethyl bromide | 〃 | 54.1 | 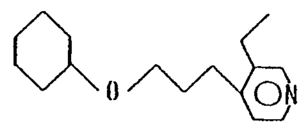 | 1-(3-ethyl-4-pyridyl)-3-cyclohexyloxy propane |

Table 3 (continued)

| Example | Alkyl halide used | Pyridine compound used | Yield (%) | Strucural formula | Name of compound |
|---|---|---|---|---|---|
| 95 | 3-(3,5,5-tri-methyloxy)-propyl bromide | 3-ethyl-4-methyl pyridine | 17.7 | 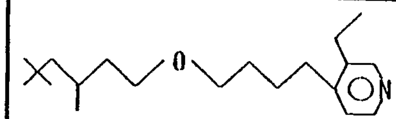 | 1-(3-ethyl-4-pyridyl)-4-(3,5,5-trimethylhexoxyl)-butane |
| 96 | 3-methyl-5-(2,2-dichloro-3,3-dimethylcyclo-propyl)-pentyl bromide | 〃 | 11.0 | 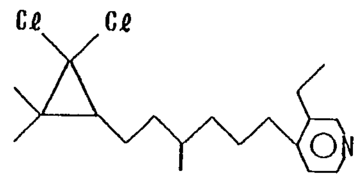 | 1-(3-ethyl-4-pyridyl)-4-methyl-6-(2,2-dichloro-3,3-dimethylcyclopropyl)-hexane |
| 97 | 3-(2-tetrahydro-pyranyl)-propyl bromide | 〃 | 27.1 | 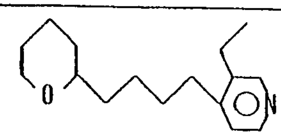 | 1-(3-ethyl-4-pyridyl)-4-(2-tetrahydropyranyl)-butane |
| 98 | 2-(2-n-hexyloxyethyl-oxy)-ethyl bromide | 〃 | 71.9 | 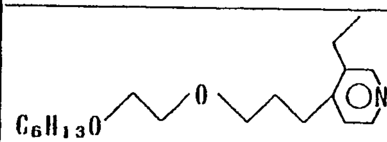 | 1-(3-ethyl-4-pyridyl)-3-(2-n-hexyloxyethyloxy)-propane |
| 99 | 2-ethyloxyethyl bromide | 〃 | 10.0 | 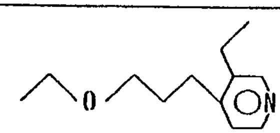 | 1-(3-ethyl-4-pyridyl)-3-ethyloxy propane |
| 100 | isobutyl bromide | 1-(3-ethyl-4-pyridyl)-3-tert-butyloxy propane | 30.7 | 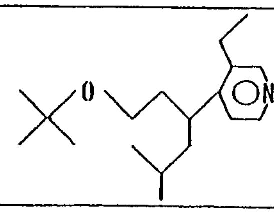 | 2-methyl-4-(3-ethyl-4-pyridyl)-6-tert-butyloxy hexane |

Table 3 (continued)

| Example | Alkyl halide used | Pyridine compound used | Yield (%) | Structural formula | Name of compound |
|---|---|---|---|---|---|
| 101 | 2-isobutyloxy-ethyl bromide | 3,4-dimethyl-5-ethyl pyridine | 10.5 | 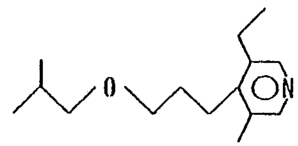 | 1-(3-ethyl-5-methyl-4-pyridyl)-3-isobutyloxy propane |
| 102 | 2-(2,2'-dichloro-1-methylcyclopropyl)-ethyl bromide | 3-ethyl-4-methyl pyridine | 17.2 | 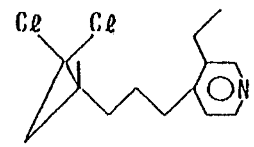 | 1-(3-ethyl-4-pyridyl)-3-(2,2-dichloro-1-methyl cyclopropyl)-propane |
| 103 | 2,2-dichloro-3,3-dimethylcyclo-propylmethyl bromide | 〃 | 69.0 | 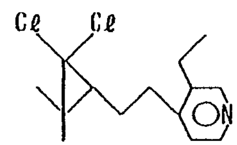 | 1-(3-ethyl-4-pyridyl)-2-(2,2-dichloro-3,3-dimethylcyclopropyl)-ethane |
| 104 | 3-(2-tert-butyloxy-ethyloxy)-propyl bromide | 〃 | 32.2 | 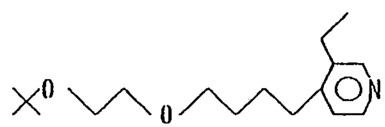 | 1-(3-ethyl-4-pyridyl)-4-(2-tert-butyloxyethyl-oxy)-butane |
| 105 | 2-isopropyloxy-ethyl bromide | 〃 | 89.2 | 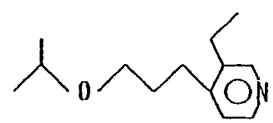 | 1-(3-ethyl-4-pyridyl)-3-isopropyloxy propane |

Table 3 (continued)

| Example | Alkyl halide used | Pyridine compound used | Yield (%) | Structural formula | Name of compound |
|---|---|---|---|---|---|
| 106 | 2-(2-norbonyloxy)-ethyl bromide | 3-ethyl-4-methyl pyridine | 38.6 | 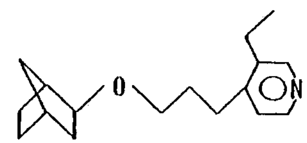 | 1-(3-ethyl-4-pyridyl)-3-(2-norbonyloxy)-propane |
| 107 | 2-cycloheptyloxy-ethyl bromide | 〃 | 22.8 | 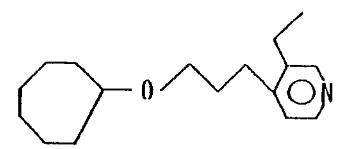 | 1-(3-ethyl-4-pyridyl)-3-cycloheptyloxy propane |
| 108 | 2-cyclopentyloxy-ethyl bromide | 〃 | 84.3 | 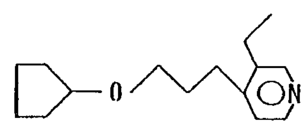 | 1-(3-ethyl-4-pyridyl)-3-cyclopentyloxy propane |

Table 4

| Example | Infrared absorption spectrum *1 ($cm^{-1}$) | Proton nuclear magnetic resonance spectrum *2 (ppm) |
|---|---|---|
| 30 | 2880~3060, 1600, 1495 | 0.89(12H,d), 1.24(3H,t), 1.0~1.9 (9H,m), 2.45~2.8(4H,m), 7.03(1H,d), 8.32(1H,d), 8.35(1H,s) |
| 31 | 2890~3080, 1608, 1500 | 0.90(6H,t), 1.24(3H,t), 1.0 ~1.7 (15H,m), 2.4~2.8(4H,m), 7.03(1H,d), 8.32(1H,d), 8.35(1H,s) |
| 32 | 2890~3090, 1602, 1480 | 0.82(9H,s), 1.20(3H,d), 1.22(3H,t), 1.0 ~1.7(6H,m), 2.68(2H,q), 2.98 (1H,q), 7.08 (1H,d), 8.35(1H,s), 8.37(1H,d) |
| 33 | 2900~2990, 1475 | 0.84(6H,t), 1.23(3H,t), 1.1 ~1.8 (11H,m), 2.5~2.8(4H,m), 7.04(1H,d), 8.32(1H,d), 8.35(1H,s) |
| 34 | 2870~2940, 1603, 1500 | 0.88(3H,t), 1.2 ~1.8(29H,m), 2.5 ~ 2.8(4H,m), 7.05(1H,d), 8.33(1H,d), 8.35(1H,s) |
| 35 | 2880~3070, 1598, 1477 | 0.89(9H,s), 1.1 ~2.0(10H,m), 2.6 ~ 2.85(3H,m), 7.06(1H,d), 8.28(1H,s), 8.29(1H,d) |
| 36 | 2880~3080, 1600, 1497 | 0.90(3H,t), 1.1 ~2.0(12H,m), 2.71 (3H,brs), 7.05(1H,d), 8.28(1H,s), 8.29(1H,d) |
| 37 | 2900~3080, 1608, 1506 | 1.24(3H,t), 1.80(3H,s), 1.5~1.9 (2H,m), 2.1 ~2.4(2H,m), 2.5~2.9 (4H,m), 7.06(1H,d), 8.33(1H,d), 8.36(1H,s) |
| 38 | 2900~3000, 1606, 1502 | 1.09(3H,t), 1.24(3H,t), 2.0~2.3(2H, m), 2.3 ~2.6(2H,m), 2.6~2.9(4H,m), 7.10(1H,d), 8.35(1H,d), 8.38(1H,d) |

Table 4 (continued)

| Example | Infrared absorption spectrum (cm⁻¹) *1 | Proton nuclear magnetic resonance spectrum (ppm) *2 |
|---|---|---|
| 39 | 2900～3100, 1652, 1604, 1501 | 1.23(3H,t), 1.5 ～1.9(2H,m), 2.0～2.35(2H,m), 2.5 ～2.85(4H,m), 4.9 ～5.2(2H,m), 5.6～6.1(1H,m), 7.05(1H,d), 8.33(1H,d), 8.36(1H,s) |
| 40 | 2900～2980, 1602, 1500 | 0.80(6H,d), 0.87(3H,d), 1.23(3H,t), 1.0 ～1.8(6H,m), 2.5～2.8(4H,m), 7.05(1H,d), 8.33(1H,d), 8.37(1H,s) |
| 41 | 2890～3060, 1600, 1497 | 0.77(3H,d), 0.83(3H,d), 0.88(3H,t), 1.23(3H,t), 1.0 ～1.8(8H,m), 2.5～2.8(4H,m), 7.05(1H,d), 8.33(1H,d), 8.36(1H,s) |
| 42 | 2890～3070, 1601, 1475 | 0.88(9H,s), 0.90(3H,d), 1.23(3H,t), 1.0 ～1.8(11H,m), 2.5 ～2.8(4H,m), 7.04(1H,d), 8.32(1H,d), 8.35(1H,s) |
| 43 | 2880～3080, 1603, 1500 | 0.86(6H,d), 1.23(3H,t), 1.1 ～1.8(11H,m), 2.5～2.8(4H,m), 7.04(1H,d), 8.32(1H,d), 8.35(1H,s) |
| 44 | 2900～3000, 1601, 1486 | 0.89(9H,s), 0.90(3H,d), 1.23(3H,t), 1.0 ～1.6(7H,m), 2.27(3H,s), 2.4～2.8(4H,m), 8.18(1H,s), 8.22(1H,s) |
| 45 | 2880～3110, 1603, 1500 | 1.19(3H,t), 1.21(3H,d), 1.4 ～1.8(15H,m), 2.69(2H,q), 2.8～3.2(1H,m), 7.17(1H,d), 8.32(1H,d), 8.33(1H,s) |
| 46 | 2860～3050, 1596, 1492 | 1.23(3H,t), 0.9 ～2.3(15H,m), 2.5 ～2.8(4H,m), 7.04(1H,d), 8.32(1H,d), 8.35(1H,s) |
| 47 | 2860～3070, 1602, 1500 | 1.23(3H,t), 1.3 ～2.1(19H,m), 2.45～2.8(4H,m), 7.04(1H,d), 8.32(1H,d), 8.35(1H,s) |

Table 4 (continued)

| Example | Infrared absorption spectrum (cm⁻¹) [*1] | Proton nuclear magnetic resonance spectrum (ppm) [*2] |
|---|---|---|
| 48 | 2880~2950, 1600, 1468 | 0.89(9H,s), 1.23(3H,t), 1.1 ~1.8 (14H,m), 2.5~3.8(4H,m), 7.06(1H,d), 8.33(1H,d), 8.36(1H,s) |
| 49 | 2870~2980, 1605, 1502 | 0.90(6H,s), 0.94(3H,d), 1.14(3H,t), 1.1 ~1.9(12H,m), 7.04(1H,d), 8.32 (1H,d), 8.35(1H,s) |
| 50 | 2860~2970, 1598, 1496 | 0.88(3H,t), 1.23(3H,t), 1.0 ~1.9 (20H,m), 2.55 ~2.8(4H,m), 7.03(1H, d), 8.32(1H,d), 8.35(1H,s) |
| 51 | 2870~3060, 1601, 1500 | 0.88(3H,t), 1.23(3H,t), 1.0 ~2.3 (19H,m), 2.45 ~2.8(4H,m), 5.41(1H, brs), 7.04(1H,d), 8.32(1H,d), 8.35 (1H,s) |
| 52 | 2870~3060, 1601, 1498 | 1.23(3H,t), 1.0 ~1.9(17H,m), 2.5 ~ 2.8(4H,m), 7.06(1H,d), 8.33(1H,d), 8.36(1H,s) |
| 53 | 2860~3050, 1596, 1468 | 0.83(9H,s), 1.32(3H,t), 1.0 ~1.9 (16H,m), 2.5~2.8(4H,m), 7.06(1H,d), 8.33(1H,d), 8.36(1H,s) |
| 54 | 2870~3080, 1602, 1500 | 0.82(3H,d), 1.22(3H,t), 1.0 ~2.0 (16H,m), 2.5~2.8(4H,m), 7.05(1H,d), 8.3(1H,d), 8.36(1H,d) |
| 55 | 2880~3050, 1603, 1497 | 1.23(3H,t), 1.4 ~2.4(13H,m), 2.5 ~ 2.8(4H,m), 5.3~5.5(2H,m), 7.03 (1H,d), 8.32(1H,d), 8.35(1H,s) |
| 56 | 2870~3060, 1600, 1496 | 1.23(3H,t), 0.9 ~2.0(15H,m), 2.5 ~ 2.8(4H,m), 7.04(1H,d), 8.32(1H,d), 8.35(1H,s) |
| 57 | 2880~3080, 1605, 1500 | 1.23(3H,t), 1.1 ~1.8(19H,m), 2.4 ~ 2.8(4H,m), 7.04(1H,d), 8.32(1H,d), 8.35(1H,s) |

Table 4 (continued)

| Example | Infrared absorption spectrum *1 (cm⁻¹) | Proton nuclear magnetic resonance spectrum *2 (ppm) |
|---|---|---|
| 58 | 2880~2950, 1598, 1492 | 1.23(3H,t), 0.9 ~1.9(13H,m), 2.5 ~ 2.8(4H,m), 7.04(1H,d), 8.32(1H,d), 8.35(1H,s) |
| 59 | 2940~3070, 1601, 1500 | 1.24(3H,t), 1.1 ~2.1(7H,m), 2.2~ 3.0(8H,m), 7.05(1H,d), 7.07(4H,s), 8.34(1H,d), 8.37(1H,s) |
| 60 | 2880~3070, 1598, 1496 | 0.87(9H,d), 1.23(3H,t), 1.0 ~1.8 (10H,m), 2.5~2.8(4H,m), 7.04(1H,d), 8.33(1H,d), 8.36(1H,s) |
| 61 | 2870~2960, 1595, 1492 | 0.7 ~1.0(9H,m), 1.23(3H,t), 1.0~ 1.8(10H,m), 2.5 ~2.8(4H,m), 7.04 (1H,d), 8.33(1H,d), 8.36(1H,s) |
| 62 | 2880~3060, 1602, 1500 | 0.71(9H,t), 1.23(3H,t), 1.0 ~1.6 (10H,m), 2.45 ~2.8(4H,m), 7.05 (1H,d), 8.32(1H,d), 8.35(1H,s) |
| 63 | 2890~2980, 1600, 1477 | 0.82(9H,s), 1.23(3H,s), 1.0 ~1.75 (5H,m), 2.45~2.8(4H,m), 7.05(1H,d), 8.33(1H,d), 8.35(1H,d) |
| 64 | 2890~3030, 1600, 1468 | 1.00(9H,d), 1.23(3H,t),1.6~2.2(4H, m), 2.5 ~2.8(4H,m), 5.2~5.6(2H,m), 7.05(1H,d), 8.32(1H,d), 8.36(1H,s) |
| 65 | 2880~2970, 1598, 1494 | 0.76(3H,s), 0.82(3H,d), 0.86(3H,t), 1.22(3H,t), 1.0 ~1.8(10H,m), 2.5 ~ 2.8(4H,m), 7.05(1H,d), 8.33(1H,d), 8.36(1H,s) |
| 66 | 2900~3090, 1610, 1504 | 0.75~1.0(9H,m), 1.24(3H,t), 1.0~ 1.8(10H,m), 2.5 ~2.8(4H,m), 7.05 (1H,d), 8.33(1H,d), 8.36(1H,s) |

Table 4 (continued)

| Example | Infrared absorption spectrum (cm⁻1) *1 | Proton nuclear magnetic resonance spectrum (ppm) *2 |
|---|---|---|
| 67 | 2870~3050, 1596, 1490 | 0.8 ~0.92 (9H,m), 1.24 (3H,t), 1.0 ~ 1.8 (12H,m), 2.5 ~2.8 (4H,m), 7.04 (1H,d), 8.33 (1H,d), 8.36 (1H,s) |
| 68 | 2900~3060, 1612, 1481 | 0.88 (9H,s), 0.92 (3H,d), 1.29 (6H,d), 1.0 ~1.7 (7H,m), 2.61 (2H,t), 3.0~ 3.4 (1H,m), 7.02 (1H,d), 8.30 (1H,d), 8.47 (1H,s) |
| 69 | 2880~2960, 1586, 1467 | 0.89 (9H,s), 0.92 (3H,d), 1.16 (6H,t), 1.0 ~1.7 (7H,m), 2.65 (4H,q), 8.22 (2H,s) |
| 70 | 2900~3080, 1603, 1500 | 0.80 (6H,t), 1.20 (3H,d), 1.22 (3H,t), 1.0 ~1.7 (11H,m), 2.67 (2H,q), 2.8 ~ 3.1 (1H,m), 7.09 (1H,d), 8.35 (1H,s), 8.37 (1H,d) |
| 71 | 2910~3000, 1605, 1490 | 0.89 (9H,s), 0.99 (3H,d), 1.1 ~1.7 (11H,m), 1.24 (3H,t), 2.5~2.8 (6H,m), 8.20 (2H,s) |
| 72 | 2870~3060, 1598, 1492 | 0.84 (6H,t), 1.24 (3H,t), 1.1 ~1.8 (11H,m), 2.5~2.8 (4H,m), 7.05 (1H,d), 8.33 (1H,d), 8.36 (1H,s) |
| 73 | 2900~3080, 1608, 1502 | 0.84 (6H,t), 0.95 (3H,d), 1.24 (3H,t), 1.1 ~1.8 (10H,m), 2.5 ~2.8 (4H,m), 7.04 (1H,d), 8.32 (1H,d), 8.35 (1H,s) |
| 74 | 2890~3070, 1603, 1479 | 0.89 (9H,s), 0.91 (3H,d), 1.23 (3H,t), 1.0 ~1.7 (9H,m), 2.62 (2H,t), 2.66 (2H,q), 7.04 (1H,d), 8.32 (1H,d), 8.35 (1H,s) |
| 75 | 2900~3060, 1599, 1480 | 0.89 (9H,s), 0.91 (3H,d), 1.25 (3H,t), 1.29 (6H,d), 1.1 ~1.6 (7H,m), 2.5~ 2.8 (4H,m), 2.95 ~3.3 (1H,m), 8.20 (1H,s), 8.33 (1H,s) |

Table 4 (continued)

| Example | Infrared absorption spectrum *1 (cm⁻¹) | Proton nuclear magnetic resonance spectrum *2 (ppm) |
|---|---|---|
| 76 | 2900~3000, 1608, 1500 | 0.96(3H,t), 0.98(3H,t), 1.23(3H,t), 1.4 ~2.3(6H,m), 2.45 ~2.8(4H,m), 5.1 ~5.4(1H,m), 7.04(1H,d), 8.32 (1H,d), 8.35(1H,s) |
| 77 | 2860~2940, 1592 | 0.83(3H,d), 1.23(3H,t), 0.9 ~1.9 (16H,m), 2.27(3H,s), 2.5~2.8(4H,m), 8.19(2H,s) |
| 78 | 2900~3060, 1601, 1472 | 1.23(3H,t), 0.9 ~2.2(15H,m), 2.27 (3H,s), 2.5 ~2.8(4H,m), 8.18(1H,s), 8.21(1H,s) |
| 79 | 2900~2950, 1601, 1495 | 1.23(3H,t), 1.1 ~1.8(25H,m), 2.5 ~ 2.8(4H,m), 7.04(1H,d), 8.32(1H,d), 8.35(1H,s) |
| 80 | 2870~2950, 1601, 1496 | 1.23(3H,t), 1.1 ~1.8(27H,m), 2.5 ~ 2.8(4H,m), 7.04(1H,d), 8.32(1H,d), 8.35(1H,s) |
| 81 | 2890~3070, 1601, 1496 | 0.77(3H,s), 0.81(6H,s), 1.24(3H,t), 1.0 ~2.1(12H,m), 2.5 ~2.8(4H,m), 7.05(1H,d), 8.31(1H,d), 8.35(1H,s) |
| 82 | 2860~3050, 1598, 1492 | 1.0 ~1.8(26H,m), 2.5 ~2.8(4H,m), 7.03(1H,d), 8.32(1H,d), 8.35(1H,s) |
| 83 | 2910~3080, 1608, 1502 | 1.24(3H,t), 1.3 ~1.9(19H,m), 2.5 ~ 2.8(4H,m), 7.05(1H,d), 8.32(1H,d), 8.35(1H,s) |
| 84 | 2890~3070, 1598, 1496 | 0.84(3H,d), 0.88(3H,s), 0.90(3H,s), 1.23(3H,t), 1.1 ~1.8(14H,m), 2.61 (2H,t), 2.65(2H,q), 7.03(1H,d), 8.32 (1H,d), 8.35(1H,s) |

Table 4 (continued)

| Example | Infrared absorption spectrum (cm-1) *1 | Proton nuclear magnetic resonance spectrum (ppm) *2 |
|---|---|---|
| 85 | 2860～3050, 1598, 1496 | 1.23(3H,t), 1.0 ～1.8(19H,m), 2.61 (2H,t), 2.65(2H,q), 7.03(1H,d), 8.32 (1H,d), 8.35(1H,s) |
| 86 | 2870～3070, 1602, 1500 | 1.23(3H,t), 1.0 ～1.8(21H,m), 2.61 (2H,t), 2.65(2H,q), 7.03(1H,d), 8.32 (1H,d), 8.35(1H,s) |
| 87 | 2890～2990, 1610, 1500 | 1.23(3H,t), 1.61(3H,s), 1.69(3H,s), 1.3 ～2.2(6H,m), 2.5～2.8(4H,m), 5.0 ～5.25(1H,m), 7.03(1H,d), 8.33(1H, d), 8.35(1H,s) |
| 88 | 2830～3000, 1612, 1502 | 0.90(3H,t), 1.23(3H,t), 1.0 ～2.1 (10H,m), 2.5～2.9(4H,m), 3.3～3.6 (4H,m), 7.05(1H,d), 8.33(1H,d), 8.36 (1H,s) |
| 89 | 2900～3070, 1500 | 0.97(6H,d), 1.21(3H,t), 1.6 ～2.1 (3H,m), 2.4 ～2.9(8H,m), 7.14(1H,d), 8.29(1H,d), 8.35(1H,s) |
| 90 | 2890～3080, 1601, 1498 | 0.94(6H,d), 1.21(3H,t), 1.24(3H,d), 1.5 ～2.1(3H,m), 2.37(2H,d), 2.70 (2H,t), 3.0 ～3.4(1H,m), 7.20(1H,d), 8.34(1H,d), 8.34(1H,s) |
| 91 | 2860～3060, 1602, 1500 | 1.22(3H,t),1.5～2.4(6H,m), 2.4～2.9 (4H,m),3.6～4.2(4H,m), 4.54(1H,brs), 7.04(1H,d), 8.30(1H,d), 8.33(1H,s) |
| 92 | 2850～3050, 1604, 1501 | 1.22(3H,t), 1.55(3H,s), 1.7 ～2.1 (6H,m), 2.6 ～2.9(8H,m), 7.15(1H,d), 8.30(1H,d), 8.35(1H,s) |
| 93 | 2890～2980, 1596, 1484 | 1.19(9H,s), 1.24(3H,t), 1.7 ～2.0 (2H,m), 2.5 ～2.8(4H,m), 3.38(2H,t), 7.08(1H,d), 8.33(1H,d), 8.36(1H,s) |

Table 4 (continued)

| Example | Infrared absorption spectrum *1 ($cm^{-1}$) | Proton nuclear magnetic resonance spectrum *2 (ppm) |
|---|---|---|
| 94 | 2860～2940, 1597, 1494 | 1.23(3H,t), 1.1 ～2.1(13H,m), 2.5 ～ 2.9(4H,m), 3.0～3.4(1H,m), 3.47 (2H,t), 7.06(3H,d), 8.33(1H,d), 8.36 (1H,s) |
| 95 | 2820～3080, 1604, 1480 | 0.88(9H,s), 0.94(3H,d), 1.23(3H,t), 1.1 ～1.8(9H,m), 2.5～2.8(4H,m), 3.3 ～3.55(4H,m), 7.05(1H,d), 8.32 (1H,d), 8.36(1H,s) |
| 96 | 2900～3070, 1614, 1503 | 0.8 ～1.0(1H,m), 1.15(3H,s), 1.23 (3H,t), 1.29(3H,d), 1.33(3H,s), 1.0 ～1.8(9H,m), 2.5～2.8(4H,m), 7.04 (1H,d), 8.32(1H,d), 8.35(1H,s) |
| 97 | 2860～3030, 1604, 1498 | 1.22(3H,t), 1.2 ～1.9(12H,m), 2.5 ～ 2.8(4H,m), 3.1～3.6(2H,m), 3.8～4.1 (1H,m), 7.03(1H,d), 8.31(1H,d), 8.34 (1H,s) |
| 98 | 2890～3080, 1608, 1497 | 0.88(3H,t), 1.23(3H,t), 1.1 ～1.7 (8H,m), 1.8 ～2.1(2H,m), 2.5～2.9 (4H,m), 3.4 ～3.6(4H,m), 3.59(4H,s), 7.06(1H,d), 8.32(1H,d), 8.35(1H,s) |
| 99 | 2830～3000, 1608, 1500 | 1.21(3H,t), 1.23(3H,t), 1.7 ～2.1 (2H,m), 2.67(2H,q), 2.71(2H,t), 3.43 (2H,t), 3.48(2H,t), 7.06(1H,d), 8.33 (1H,d), 8.36(1H,d) |
| 100 | 2900～3040, 1602, 1500 | 0.85(3H,d), 0.88(3H,d), 1.10(9H,s), 1.25(3H,t), 1.4 ～1.6(3H,m), 1.7～ 2.0(2H,m), 2.73(2H,q), 3.0～3.3 (3H,m), 7.08(1H,d), 8.36(1H,d), 8.37(1H,s) |

Table 4 (continued)

| Example | Infrared *1 absorption spectrum ($cm^{-1}$) | *2 Proton nuclear magnetic resonance spectrum (ppm) |
|---|---|---|
| 101 | 2890~2980, 1592, 1473 | 0.93(6H,d), 1.23(3H,d), 1.6 ~2.0 (3H,m), 2.29(3H,s), 2.67(2H,q), 2.71 (2H,t), 3.21(2H,d), 3.47(2H,t), 8.19 (1H,s), 8.22(1H,s) |
| 102 | 2990~3080, 1603, 1500 | 1.23(3H,t), 1.34(3H,s), 1.2 ~1.4 (2H,m), 1.5 ~1.9(4H,m), 2.5~2.8 (4H,m), 7.07(1H,d), 8.34(1H,d), 8.37 (1H,s) |
| 103 | 2900~3080, 1614, 1503 | 1.12(3H,s), 1.26(3H,t), 1.33(3H,s), 1.1 ~1.4(1H,m), 1.6~1.9(2H,m), 2.5 ~2.9(4H,m), 7.06(1H,d), 8.36 (1H,d), 8.39(1H,s) |
| 104 | 2880~2980, 1598, 1490 | 1.19(9H,s), 1.23(3H,t), 1.6 ~1.8 (4H,m), 2.5 ~2.8(4H,m), 3.35 ~3.6 (6H,m),7.06(1H,d), 8.32(1H,d), 8.35 (1H,s) |
| 105 | 2900~3000, 1605, 1500 | 1.17(6H,d), 1.24(3H,t), 1.7 ~2.0 (2H,m), 2.55~2.8(4H,m), 3.35 ~3.7 (3H,m), 7.06(1H,d), 8.33(1H,d), 8.37 (1H,s) |
| 106 | 2900~3080, 1607, 1503 | 1.15(3H,t), 1.1 ~2.1(12H,m), 2.5 ~ 2.8(4H,m), 3.38(2H,t), 3.6~3.9 (1H,m), 7.06(1H,d), 8.33(1H,d), 8.36 (1H,s) |
| 107 | 2880~3080, 1606, 1496 | 1.23(3H,t), 1.1 ~2.0(14H,m), 2.4 ~ 2.8(4H,m), 3.3~3.6(3H,m), 7.06 (1H,d), 8.32(1H,d), 8.35(1H,s) |
| 108 | 2900~3060, 1603, 1500 | 1.23(3H,t), 1.4 ~2.0(10H,m), 2.5 ~ 2.8(4H,m), 3.40(2H,t), 3.8~4.0 (1H,m), 7.05(1H,d), 8.33(1H,d), 8.36 (1H,s) |

*1 by the potassium bromide disc method

*2 internal standard: tetramethyl silane

Examples 109 to 111.

An alcoholic compound in a specified amount as indicated in Table 5 was admixed with 4 ml of 65% sulfuric acid and heated at 100° C for 2 hours. After cooling, water was added and it was converted alkaline with sodium carbonate followed by extraction with ethyl acetate. Thereafter, the ethyl acetate layer was dried over anhydrous sodium sulfate followed by removal of the solvent by distillation under reduced pressure to give a compound. The structural formulas, yields, analytical results and the like of the thus obtained compounds are shown in Tables 5 and 6.

Table 5

| Example | Alcoholic compound used | Amount of alcoholic compound used (g) | Yield (g) | Yield (%) | Values of elementary analysis* (%) | | |
|---|---|---|---|---|---|---|---|
| | | | | | C | H | N |
| 109 | 2-cyclohexyl-1-(3-ethyl-4-pyridyl)-2-propanol | 2.0 | 0.26 | 14 | 84.01 (83.79) | 9.99 (10.11) | 6.00 (6.11) |
| 110 | 1-(3-ethyl-4-pyridyl)-2-methyl-2-dodecanol | 2.5 | 1.21 | 51 | 83.19 (83.56) | 11.96 (11.57) | 4.85 (4.79) |
| 111 | 3,4-dimethyl-2-(3-ethyl-4-pyridyl)-3-pentanol | 1.8 | 0.25 | 15 | 82.60 (82.70) | 10.53 (10.41) | 6.88 (6.89) |

* calculated value shown in brackets

Table 6

| Example | Structural formula | Chemical formula | Name of compound | Infrared absorption spectrum ($cm^{-1}$) *1 | Proton nuclear magnetic resonance spectrum (ppm) *2 |
|---|---|---|---|---|---|
| 109 | 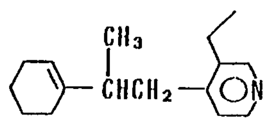 | $C_{16}H_{23}N$ | 2-(1-cyclohexenyl)-1-(3-ethyl-4-pyridyl)-propane | 2880~3000<br>1600, 1500 | 0.99 (3H, d), 1.24 (3H, t),<br>1.4 ~2.9 (13H, m),<br>5.35 (1H, br s) 7.14 (1H, d),<br>8.29 (1H, d), 8.36 (1H, s) |
| 110 | 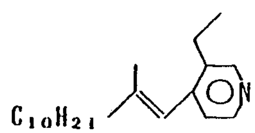 | $C_{20}H_{33}N$ | (E)-1-(3-ethyl-4-pyridyl)-2-methyl-1-dodecene | 2880~3000<br>1662, 1603<br>1498 | 0.88 (3H, t), 1.16 (3H, t),<br>1.0 ~1.7 (16H, m), 1.70 (3H, d),<br>2.20 (2H, t), 2.60 (2H, q),<br>6.21 (1H, br s), 7.01 (1H, d),<br>8.35 (1H, d), 8.39 (1H, s) |
| 111 | 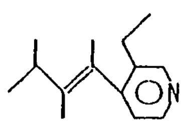 | $C_{14}H_{21}N$ | (E)-3,4-dimethyl-2-(3-ethyl-4-pyridyl)-2-pentene | 2890~3050<br>1600, 1497 | 1.14 (3H, t), 1.22 (3H, d),<br>1.31 (3H, d), 1.66 (3H, s),<br>1.82 (3H, d), 2.35~2.8 (3H, m),<br>7.17 (1H, d), 8.33 (1H, s),<br>8.38 (1H, d) |

*1 and *2: the same as in Table 2

Examples 112 to 114.

In Example 109, a compound was obtained by conducting the same procedure as in Example 109 excepting the use of the alcoholic compound indicated in Tables 7 and 8. The structural formulas, yields and the like of the thus obtained compounds are shown in Tables 7 and 8.

Table 7

| Example | Alcohol compound used | Yield (%) | Structural formula | Name of compound |
|---|---|---|---|---|
| 112 | 2-(3-ethyl-4-pyridyl)-3-methyl-3-pentanol | 1.3 | 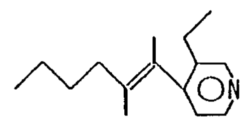 | (E)-2-(3-ethyl-4-pyridyl)-3-methyl-2-heptene |
| 113 | 1-(3-ethyl-4-pyridyl)-2-methyl-2-tridecanol | 1.9 | 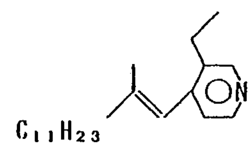 | (E)-1-(3-ethyl-4-pyridyl)-2-methyl-1-tridecene |
| 114 | 1-[3-(1-hydroxyethyl)-4-pyridyl]-4,6,6-trimethyl heptane | 85.5 | 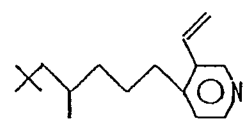 | 1-[3-(1-vinyl)-4-pyridyl]-4,6,6-trimethyl heptane |

Table 8

| Example | Infrared absorption spectrum *1 ($cm^{-1}$) | Proton nuclear magnetic resonance spectrum *2 (ppm) |
|---|---|---|
| 112 | 2880~3060, 1597, 1495 | 0.74(3H,t), 1.17(3H,t), 0.9 ~1.6(4H,m), 1.81(3H,s), 1.86(3H,s), 1.6 ~1.9(2H,m), 2.55(2H,q), 6.91(1H,d), 8.32(1H,d), 8.44(1H,s) |
| 113 | 2870~2980, 1660, 1600, 1497 | 0.88(3H,t), 1.0 ~1.6(21H,m), 1.90(3H,d), 1.9 ~2.2(2H,m), 2.59(2H,q), 6.19(1H,brs), 6.99(1H,d), 8.35(1H,d), 8.38(1H,s) |
| 114 | 2880~3100, 1637, 1596, 1475 | 0.88(9H,s), 0.90(3H,d), 1.0 ~1.8(7H,m), 2.63(2H,t), 5.39(1H,dd), 5.68(1H,dd), 6.90(1H,dd), 7.04(1H,d), 8.36(1H,d), 8.60(1H,s) |

*1 by the potassium bromide disc method

*2 internal standard: tetramethyl silane

Example 115.

The compound obtained in Example 2 was reacted with oxalic acid to give an oxalic acid salt of 3-ethyl-4-(3,5,5-trimethylhexyl) pyridine. This compound had a melting point of 101.6 to 103.2 °C.

Example 116.

The compound obtained in Example 14 was reacted with hydrogen chloride to give a hydrochloric acid salt of 3-ethyl-4-(5,5-dimethylhexyl) pyridine. This compound had a melting point of 184.6 to 185.2 °C.

Examples 117 to 121.

Into a 100 ml three-necked flask were introduced 3.27 g (15.8 mM) of 1-(3-ethyl-4-pyridyl)-5-hexanol and 3 ml of thionyl chloride werè added thereto dropwise while it was agitated under chilling with ice. Agitation was performed for 1 hour under chilling with ice and agitation was then performed for 30 minutes at room temperature followed by the addition of toluene and removal of toluene and excess of thionyl chloride by distillation under reduced pressure. The residue was admixed with water and, after conversion into alkalinity with sodium carbonate, extracted with ethyl acetate. After drying over anhydrous sodium sulfate, ethyl acetate was distilled off and purification was conducted by silica gel column chromatography to give 0.57 g (yield 16%) of an oily compound. compounds of Examples 118 to 121 were obtained in the same manner. The structural formulas, yields and the like of the thus obtained compounds are shown in Tables 9 and 10.

Table 9

| Example | Alcohol compound used | Yield (%) | Structural formula | Name of compound |
|---|---|---|---|---|
| 117 | 1-(3-ethyl-4-pyridyl)-5-hexanol | 16.0 | 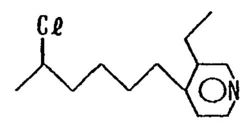 | 1-(3-ethyl-4-pyridyl)-5-chlorohexane |
| 118 | 2-(3-ethyl-4-pyridyl)-3-heptanol | 6.8 | 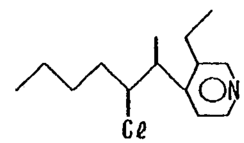 | 2-(3-ethyl-4-pyridyl)-3-chloroheptane |
| 119 | 1-(3-ethyl-4-pyridyl)-6-methyl-4-heptanol | 19.0 | 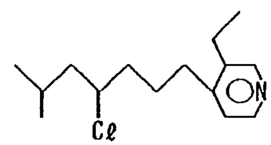 | 1-(3-ethyl-4-pyridyl)-4-chloro-6-methyl heptane |
| 120 | 1-(3-ethyl-4-pyridyl)-4-octanol | 30.0 | 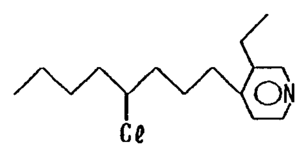 | 1-(3-ethyl-4-pyridyl)-4-chlorooctane |
| 121 | 1-(3-ethyl-4-pyridyl)-4-cyclohexyl-4-butanol | 27.0 | 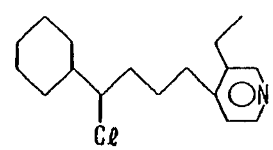 | 1-(3-ethyl-4-pyridyl)-4-chloro-4-cyclohexyl butane |

Table 10

| Example | Infrared absorption spectrum (cm$^{-1}$) *1 | Proton nuclear magnetic resonance spectrum (ppm) *2 |
|---|---|---|
| 117 | 2900~3000, 1610, 1504 | 1.21(3H,t), 1.49(3H,d), 1.4 ~1.9(6H,m), 2.55 ~2.85 (4H,m), 3.9 ~4.3(1H,m), 7.13(1H,d), 8.29(1H,d), 8.29(1H,d), 8.34(1H,s) |
| 118 | 2890~2980, 1600, 1497 | 0.91(3H,t), 1.24(3H,t), 1.31(3H,d), 1.3 ~1.9(6H,m), 2.5 ~2.9(4H,m), 3.2~3.55 (1H,m), 4.06(1H,td), 7.18 (1H,d), 8.40(1H,d), 8.41(1H,s) |
| 119 | 2880~3060, 1600, 1493 | 0.89(3H,d), 0.92(3H,d), 1.22(3H,t), 1.4 ~2.0(7H,m), 2.5 ~2.8(4H,m), 3.7~4.2 (1H,m), 7.05(1H,d), 8.33 (1H,d), 8.37(1H,s) |
| 120 | 2880~3060, 1600, 1498 | 0.90(3H,t), 1.23(3H,t), 1.3 ~2.0(10H,m), 2.5 ~2.8 (4H,m), 3.8 ~4.1(1H,m), 7.06(1H,d), 8.33(1H,d), 8.37(1H,s) |
| 121 | 2890~2960, 1611, 1507 | 1.23(3H,t), 1.3 ~2.0(15H,m), 2.5 ~2.8(4H,m), 3.6~4.0 (1H,m), 7.06(1H,d), 8.32 (1H,d), 8.36(1H,s) |

*1 by the potassium bromide disc method

*2 internal standard: tetramethyl silane

Examples 122 to 130.

Into a 100 ml three-necked flask were introduced 0.15 g (3.6 m moles) of 60% sodium hydride and 10 ml of dimethyl formamide and, under an atmosphere of nitrogen, a dimethyl formamide solution of 0.50 g (3.6 m moles) of 3-ethyl-4-hydroxymethyl pyridine and a dimethyl formamide solution of 0.76 g (3.6 m moles) of 3,5,5-trimethylhexyl bromide were added dropwise thereto. After agitation for 3.5 hours at room temperature, the reaction mixture was poured into ice water and extracted with ethyl ether. The ethyl ether layer was washed with saturated aqueous solution of sodium chloride followed by drying over anhydrous

sodium sulfate. The solvent was removed by distillation under reduced pressure followed by purification by the silica gel column chromatography to give 0.5 g (yield 52%) of an oily material. Compounds of Examples 123 to 130 were obtained in the same manner. The structural formulas, yields and the like of the thus obtained compounds are shown in Tables 11 and 12.

Table 11

| Example | Alkyl halide used | Pyridine compound used | Yield (%) | Structural formula | Name of compound |
|---|---|---|---|---|---|
| 122 | 3,5,5-trimethyl-hexyl bromide | 3-ethyl-4-hydroxymethyl pyridine | 52.0 | 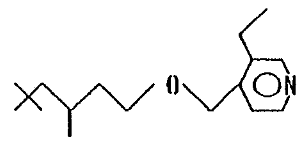 | 1-(3-ethyl-4-pyridyl)-1-(3,5,5-trimethyloxy)-methane |
| 123 | 2-propenyl bromide | 〃 | 45.5 | 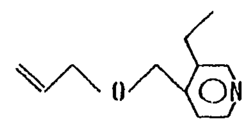 | 1-(3-ethyl-4-pyridyl)-1-(2-propenyloxy)-methane |
| 124 | 2,4,4-trimethyl-pentyl bromide | 〃 | 8.1 | 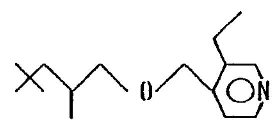 | 1-(3-ethyl-4-pyridyl)-1-(2,4,4-trimethyl-pentyloxy)-methane |
| 125 | 2-tert-butyloxy-ethyl bromide | 〃 | 20.3 | 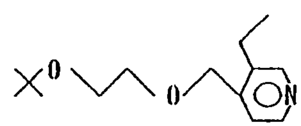 | 1-(3-ethyl-4-pyridyl)-1-(2-t-butyloxy-ethyloxy)-methane |
| 126 | 3,3-dimethyl-butyl bromide | 〃 | 30.2 | 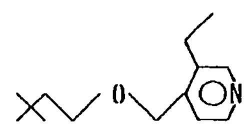 | 1-(3-ethyl-4-pyridyl)-1-(3,3-dimethyl-butyloxy)-methane |

Table 11 (continued)

| Example | Alkyl halide used | Pyridine compound used | Yield (%) | Structural formula | Name of compound |
|---|---|---|---|---|---|
| 127 | 4-methylpentyl bromide | 3-ethyl-4-hydroxyethyl pyridine | 66.7 | 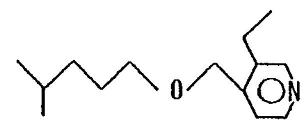 | 1-(3-ethyl-4-pyridyl)-1-(4-methylpentyloxy)-methane |
| 128 | 3-methylbutyl bromide | 3-ethyl-4-(1-hydroxy)-ethyl pyridine | 79.3 | 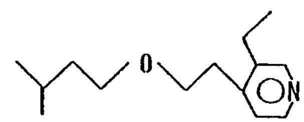 | 1-(3-ethyl-4-pyridyl)-2-(3-methylbutyloxy)-ethane |
| 129 | Cyclohexyl-methyl bromide | 〃 | 16.7 | 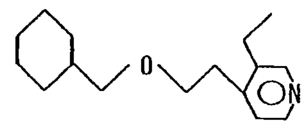 | 1-(3-ethyl-4-pyridyl)-2-cyclohexylmethyloxy ethane |
| 130 | 〃 | 3-ethyl-4-(1-hydroxy)-ethyl pyridine | 10.3 | 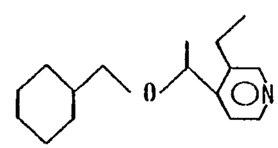 | 2-(3-ethyl-4-pyridyl)-3-cyclohexyloxy propane |

Table 12

| Example | Infrared absorption spectrum ($cm^{-1}$) *1 | Proton nuclear magnetic resonance spectrum (ppm) *2 |
|---|---|---|
| 122 | 2880～2970, 1600, 1473 | 0.80(9H,s), 0.94(3H,d), 1.23(3H,t),1.1～1.8(5H,m), 2.64(2H,q), 3.54(2H,t), 4.51 (2H,s), 7.33(1H,d),8.40(1H,s),8.43(1H,d) |
| 123 | 2900～3110, 1663, 1612, 1500 | 1.24(3H,t), 2.64(2H,q), 4.08(2H,dt), 4.55(2H,s),5.1～5.5(2H,m),5.75～6.2(1H, m), 7.35(1H,d), 8.41(1H,s), 8.44(1H,d) |
| 124 | 2900～3050, 1608, 1481 | 0.91(9H,s), 1.01(3H,d), 1.24(3H,t), 1.1 ～1.4(2H,m), 1.7～2.0(1H,m), 2.64 (2H,q), 3.1 ～3.5(2H,m), 4.51(1H,s), 7.34(1H,d),8.40(1H,s), 8.43(1H,d) |
| 125 | 2880～3020, 1600 | 1.22(9H,s), 1.23(3H,t), 2.65(2H,q), 3.55 ～3.7(4H,m), 4.62(2H,s), 7.37(1H,d), 8.40(1H,s), 8:43(1H,d) |
| 126 | 2890～3040, 1606, 1478 | 0.94(9H,s), 1.24(3H,t), 1.60(2H,t), 2.64(2H,q), 3.59(2H,t), 4.51(2H,s), 7.33(1H,d), 8.40(1H,s), 8.43(1H,d) |
| 127 | 2810～3050, 1602 | 0.90(6H,d), 1.24(3H,t),1.1～1.85(5H,m), 2.64(2H,q), 3.51(2H,t), 4.52(2H,s), 7.32(1H,d), 8.43(1H,d), 8.46(1H,s) |
| 128 | 2810～3040, 1600, 1597 | 0.89(6H,d), 1.23(3H,t), 1.3 ～1.8(3H,m), 2.68(2H,q), 2.89(2H,t), 3.45(2H,t), 3.63 (2H,t), 7.09(1H,d), 8.34(1H,d), 8.37 (1H,s) |
| 129 | 2880～3050, 1608, 1500 | 1.23(3H,t),1.4～1.9(11H,m), 2.69(2H,q), 2.89(2H,t), 3.23(2H,d), 3.62(2H,t), 7.10 (1H,d), 8.34(1H,d), 8.37(1H,s) |
| 130 | 2890～3000, 1608, 1500 | 1.25(3H,t), 1.39(3H,d),0.8～1.9(11H,m), 2.66(2H,q), 3.06(2H,d), 4.59(1H,q), 7.33 (1H,d), 8.38(1H,s), 8.44(1H,d) |

*1 by the potassium bromide disc method

*2 internal standard: tetramethyl silane

Examples 131 and 132.

Into a 100 ml three-necked flask were introduced 0.64 g (16.1 m moles) of 60% sodium hydride and 10 ml of dimethyl formamide and, under an atmosphere of nitrogen, a dimethyl formamide solution of 2.10 g (16.1 m moles) of 2,4,4-trimethyl pentanol and a dimethyl formamide solution of 3.00 g (16.1 m moles) of 4-bromo-3-ethyl pyridine were added dropwise thereto. After agitation for 1.5 hours at 100°c, the reaction mixture was poured into ice water and extracted with ethyl acetate. The ethyl acetate layer was washed with a saturated aqueous solution of sodium chloride followed by drying over anhydrous sodium sulfate. After removal of the solvent by distillation under reduced pressure, purification was performed by the silica gel

column chromatography to give 0.64 g (yield 17.0%) of an oily material. The compound of Example 132 was obtained in the same manner. The structural formulas, yields and the like of the thus obtained compounds are shown in Tables 13 and 14.

Table 13

| Example | Alcoholic compound used | Yield (%) | Structural formula | Name of compound |
|---|---|---|---|---|
| 131 | 2,4,4-trimethyl pentanol | 17.0 | 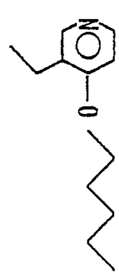 | 3-ethyl-4-(2,4,4-trimethylpentyloxy)-pyridine |
| 132 | 3,5,5-trimethyl hexanol | 25.6 | 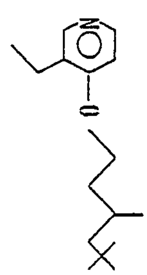 | 3-ethyl-4-(3,5,5-trimethylhexyloxy)-pyridine |

Table 14

| Example | Infrared absorption spectrum *1 (cm$^{-1}$) | Proton nuclear magnetic resonance spectrum *2 (ppm) |
|---|---|---|
| 131 | 2900～3060, 1602, 1511 | 0.95(9H,s), 1.03(3H,d), 1.13(3H,t), 1.0 ～1.6 (2H,m), 1.9 ～2.2(1H,m), 2.63(2H,q), 3.6 ～3.9 (2H,m), 6.69(1H;d), 8.25 (1H,s), 8.32(1H,d) |
| 132 | 2900～3060, 1605, 1513 | 0.91(9H,s), 1.00(3H,d), 1.20(3H,t), 1.1 ～1.9 (5H,m), 2.62(2H,q), 4.03 (2H,t), 6.73(1H,d), 8.25 (1H,s), 8.32(1H,d) |

*1 by the potassium bromide disc method

*2 internal standard: tetramethyl silane

Examples 133 to 140.

Into a 100 ml eggplant-like flask were taken 10 ml of n-butanol to which 75 mg (3.26 m moles) of sodium were added and dissolved followed by the addition of 0.5 g (2.72 m moles) of 1-chloro-3-(3-ethyl-4-pyridyl) propane and heating under reflux for 3 hours. After completion of the reaction, water was added and the solvent was removed by distillation under reduced pressure followed by extraction with ethyl acetate. The ethyl acetate layer was washed twice with 30 ml of a 5% hydrochloric acid and the aqueous solution was rendered alkaline with sodium carbonate followed by extraction with diethyl ether and washing with a saturated aqueous solution of sodium chloride followed by drying over anhydrous sodium sulfate. After removal of the solvent by distillation under reduced pressure, purification was performed by the silica gel column chromatography to give 0.6 g (yield 99.6%) of an oily compound. The compounds of Examples 134 to 140 were obtained in the same manner. The structural formulas, yields and the like of the thus obtained compounds are shown in Tables 15 and 16.

Table 15

| Example | Alcohol used | Pyridine compound used | Yield (%) | Structural formula | Name of compound |
|---|---|---|---|---|---|
| 133 | n-butanol | 1-chloro-3-(3-ethyl-4-pyridyl)-propane | 99.6 | 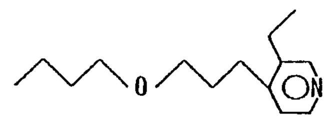 | 1-(3-ethyl-4-pyridyl)-3-n-butyloxy propane |
| 134 | n-propanol | 〃 | 88.6 | 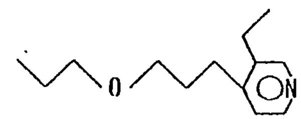 | 1-(3-ethyl-4-pyridyl)-3-n-propyloxy propane |
| 135 | Neopentyl alcohol | 〃 | 41.4 | 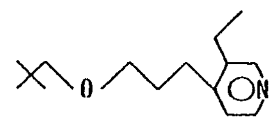 | 1-(3-ethyl-4-pyridyl)-3-(2,2-dimethyl-propyloxy)-propane |
| 136 | isoamyl alcohol | 〃 | 76.8 | 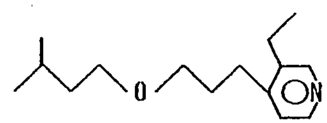 | 1-(3-ethyl-4-pyridyl)-3-(3-methylbutyloxy)-propane |
| 137 | sec-amyl alcohol | 〃 | 41.3 | 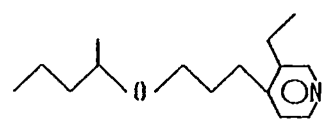 | 1-(3-ethyl-4-pyridyl)-3-(1-methylbutyloxy)-propane |

Table 15 (continued)

| Example | Alcohol used | Pyridine compound used | Yield (%) | Structural formula | Name of compound |
|---|---|---|---|---|---|
| 138 | isopropanol | 1-chloro-4-(3-ethyl-4-pyridyl)-propane | 64.3 | 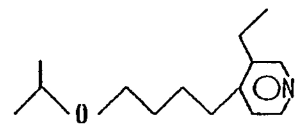 | 1-(3-ethyl-4-pyridyl)-4-isopropyloxy butane |
| 139 | 2-ethyl butanol | 1-chloro-3-(3-ethyl-4-pyridyl)-propane | 76.4 | 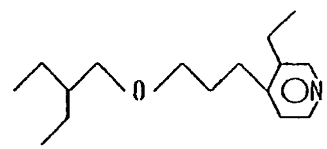 | 1-(3-ethyl-4-pyridyl)-3-(2-ethylbutyloxy)-propane |
| 140 | 2-methyl butanol | 〃 | 67.3 | 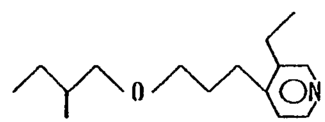 | 1-(3-ethyl-4-pyridyl)-3-(2-methylbutyloxy)-propane |

Table 16

| Example | Infrared absorption spectrum *1 ($cm^{-1}$) | Proton nuclear magnetic resonance spectrum *2 (ppm) |
|---|---|---|
| 133 | 2820~2980, 1603, 1494 | 0.93(3H,t), 1.25(3H,t), 1.1~2.0(6H,m), 2.5 ~2.8(4H,m), 3.3~3.8(4H,m), 7.06(1H,d), 8.33(1H,d), 8.36(1H,d) |
| 134 | 2890~3100, 1604, 1500 | 0.93(3H,t), 1.23(3H,t), 1.4 ~2.0(4H,m), 2.5 ~2.8(4H,m), 3.39(2H,t), 3.45(2H,t), 7.07(1H,d), 8.33(1H,d), 8.37(1H,s) |
| 135 | 2900~3000, 1608, 1500 | 0.93(9H,s), 1.23(3H,t), 1.7 ~2.1(2H,m), 2.55~2.85(4H,m), 3.07(2H,s), 3.44(2H,t), 7.06(1H,d), 8.33(1H,d), 8.36(1H,s) |
| 136 | 2820~3040, 1604, 1499 | 0.92(6H,d), 1.23(3H,t), 1.4~2.0(5H,m), 2.70(2H,q), 2.71(2H,t), 3.44(4H,t), 7.06(1H,d), 8.33(1H,d), 8.36(1H,s) |
| 137 | 2900~3050, 1606, 1500 | 0.87(3H,t), 1.13(3H,d), 1.23(3H,t), 1.3 ~2.0(6H,m), 2.5~2.8(4H,m), 3.2~3.7 (3H,m), 7.06(1H,d), 8.33(1H,d), 8.36(1H,s) |
| 138 | 2870~3060, 1597, 1486 | 1.14(6H,d), 1.23(3H,t), 1.55~1.9(4H,m), 2.62(2H,t), 2.66(2H,q), 3.4 ~3.7(3H,m), 7.05(1H,d), 8.32 (1H,d), 8.35(1H,s) |
| 139 | 2810~2980, 1600, 1496 | 0.89(6H,t), 1.23(3H,t), 1.2~1.55(4H,m), 1.7 ~2.0(3H,m), 2.5~2.8(4H,m), 3.35 (2H,d), 3.43(2H,t), 7.06(1H,d), 8.33 (1H,d), 8.36(1H,s) |
| 140 | 2800~2960, 1597, 1488 | 1.91(3H,d), 0.95(3H,t), 1.23(3H,t), 1.4~2.0(5H,m), 2.67(2H,q), 2.72(2H,t), 3.23 (2H,d), 3.43(2H,t), 7.06(1H,d), 8.33 (1H,d), 8.36(1H,s) |

*1 by the potassium bromide disc method

*2 internal standard: tetramethyl silane

Examples 141 to 143.

Into a 100 ml three-necked flask were introduced 0.19 g (4.7 m moles) of 60% sodium hydride and 10 ml of dimethyl formanide and, under an atmosphere of nitrogen, a dimethyl formamide solution of 0.70 g (4.3 m moles) of 3-ethyl-4-pyridyl methyl ketone oxime and a dimethyl formamide solution of 0.97 g (4.7 m moles) of 3,5,5-trimethylhexyl bromide were added dropwise thereto. After agitation for 1 hour at 100 °C, the reaction mixture was poured into ice water and extracted with ethyl acetate. The ethyl acetate layer was

washed with a saturated aqueous solution of sodium chloride followed by drying over anhydrous sodium sulfate. After removal of the solvent by distillation under reduced pressure, purification was performed by the silica gel column chromatography to obtain 0.97 g (yield 64.0%) of an oily material. The compounds of Examples 142 and 143 were obtained in the same manner. The structural formulas, yields and the like of the thus obtained compounds are shown in Tables 17 and 18.

Table 17

| Example | Halide used | Oxime used | Yield (%) | Structural formula | Name of compound |
|---|---|---|---|---|---|
| 141 | 3,5,5-trimethyl-hexyl bromide | 3-ethyl-pyridyl methyl ketone oxime | 64.0 | 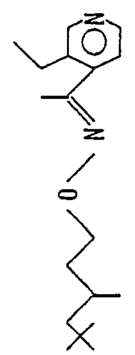 | O-(3,5,5-trimethyl-hexyl)-(3-ethyl-4-pyridyl)-methyl ketone oxime |
| 142 | benzyl bromide | 〃 | 47.4 | 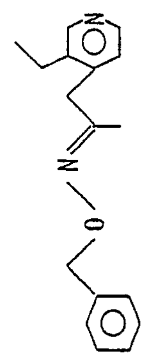 | O-benzyl(3-ethyl-4-pyridylmethyl)-methyl ketone oxime |
| 143 | 2-brom-4-(3-ethyl-4-pyridyl)-butane | 2-norborna-none oxime | 24.6 | 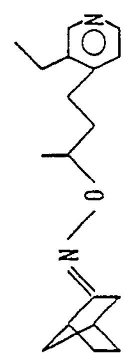 | O-[1-(3-ethyl-4-pyridyl)-3-methyl]-butyl]-2-norbornanone oxime |

Table 18

| Example | Infrared absorption spectrum *1 (cm⁻¹) | Proton nuclear magnetic resonance spectrum *2 (ppm) |
|---|---|---|
| 141 | 2900~2980, 1600, 1478 | 0.91(9H,s), 0,98(3H,d), 1.23(3H,t), 1.3 ~1.8(5H,m), 2.18(3H,s), 2.75(2H,q), 4.20(2H,t), 7.10(1H,d), 8.44(1H,d), 8.49(1H,s) |
| 142 | 2890~3100, 1598, 1502 | 1.16(3H,t), 1.78(3H,s), 2.63(2H,q), 3.49(2H,s), 5.11(2H,s), 6.98(1H,d), 7.34(5H,s), 8.35(1H,d), 8.38(1H,s) |
| 143 | 2910~3000, 1608, 1502, | 1.21(3H,t), 1.23(3H,d), 1.2 ~2.2(10H,m), 2.3 ~2.9 (6H,m), 3.9 ~4.3(1H,m), 7H(1H,d), 8.27(1H,d), 8.33(1H,s) |

*1 by the potassium bromide disc method

*2 internal standard: tetramethyl silane

Example 144.

Into a 100 ml three-necked flask were introduced 1.53 g (5.8 m moles) of triphenyl phosphine and 15 ml of acetonitrile and an acetonitrile solution of 0.35 ml (6.9 m moles) of bromine was added thereto and further an acetonitrile solution of 1.1 g (5.3 m moles) of 6-(3-ethyl-4-pyridyl) 2-hexanol was added dropwise. After agitation for 1 hour at room temperature, the reaction was completed by heating up to 60 °C. This was admixed with a 10% aqueous solution of sodium hydroxide, extracted with hexane and dried over anhydrous sodium sulfate. The solvent was removed by distillation under reduced pressure to give 1.27 g of an oily material. This was purified by the silica gel column chromatography to give 0.96 g (yield 66.9%) of oily 1-(3-ethyl-4-pyridyl)-5-bromohexane. The structural formula, IR and NMR are shown below.

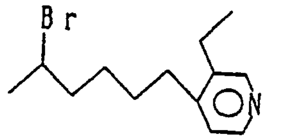

IR : 2890 to 3050, 1602, 1500
NMR: 1.24 (3H, t), 1.71 (3N, d), 1.5 to 2.0 (6H, m), 2.5 to 2.8 (4H, m), 3.95 to 4.45 (1H, m), 7.04 (1H, d), 8.33 (1H, d), 8.36 (1H, s)

Example 145.

Into a 100 ml three-necked flask were introduced 1.1 g (5.3 m moles) of 6-(3-ethyl-4-pyridyl) 2-hexanol and 20 ml of diethyl ether and, under an atmosphere of nitrogen, a diethyl ether solution of 2.7 g (6.3 m moles) of hexafluoropropene diethyl amine was added dropwise thereto. The reaction was completed by agitating for 2 hours under chilling with ice. The reaction mixture was poured into ice water, admixed with sodium hydrogen carbonate, extracted with ethyl acetate and washed with a saturated aqueous solution of sodium chloride followed by drying over anhydrous sodium sulfate. The solvent was removed by distillation under reduced pressure to give 0.82 g of an oily material. This was purified by the silica gel column chromatography to give 0.54 g (yield 48.6%) of oily 1-(3-ethyl-4-pyridyl)-5-fluorohexane. The structural formula, IR and NMR are shown below.

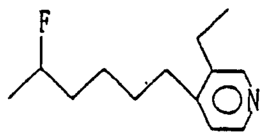

IR ; 2900 to 3090, 1610, 1508
NMR: 1.23 (3N, t), 1.32 (3H, 4d), 1.25 to 1.9 (6N, m), 2.5 to 2.8 (4H, m), 4.2 to 5.1 (1H, m), 7.04 (1H, d), 8.33 (1H, s), 8.36 (1H, s)

Example 146.

Into a 100 ml eggplant-formed flask were taken 5 ml of neopentyl alcohol and 96 mg (4.17 m moles) of sodium were added thereto and dissolved followed by the addition of 370 mg (2.78 m moles) of 3-ethyl-4-vinyl pyridine and heating under reflux for 6 hours. After completion of the reaction, water was added and the solvent was removed by distillation under reduced pressure followed by extraction with ethyl acetate. The ethyl acetate layer was washed twice with 20 ml of a 5% hydrochloric acid and the aqueous solution was rendered alkaline with sodium carbonate followed by extraction with diethyl ether and washing with a saturated aqueous solution of sodium chloride fcllowed by drying over anhydrous sodium sulfate. The solvent was removed by distillation under reduced pressure followed by purification by the silica gel column chromatography to give 270 mg (yield 44.0%) of oily 1-(3-ethyl-4-pyridyl)-2-(2,2-dimethylpropyloxy) ethane. The structural formula, IR and NMR are shown below.

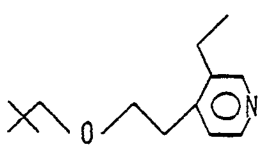

IR : 2900 to 2990, 1608, 1500
NMR: 0.87 (9H, s), 1.24 (3H, t), 2.70 (2H, q), 2.89 (2H, t), 3.06 (2H, s), 3.63 (2H, t), 7.12 (1H, d), 8.33 (1H, d), 8.36 (1H, s)

Example 147.

Ten ml of methylene chloride were admixed with 0.95 g (7.8 m moles) of 4-amino-3-ethyl pyridine and 0.79 g (7.8 m moles) of triethyl amine and, with agitation under chilling with ice, a methylene chloride solution of 1.24 g (7.0 m moles) of 3,5,5-trimethyl hexanoic acid chloride was added dropwise. Agitation for 2 hours at room temperature was followed by heating under reflux for 2 hours. Methylene chloride was removed by distillation under reduced pressure followed by the addition of an aqueous solution of sodium hydroxide and extraction with ethyl ether. The ethyl ether layer was washed twice with 30 ml of a 5% hydrochloric acid and the aqueous solution was rendered alkaline with sodium carbonate followed by extraction with ethyl ether and washing with a saturated aqueous solution of sodium chloride followed by drying over anhydrous sodium sulfate. The solvent was removed by distillation under reduced pressure followed by purification by the silica gel column chromatography to give 0.54 g (yield 29.0%) of oily N-(3-methyl-4-pyridyl)-3,5,5-trimethyl hexanoic acid amide. The structural formula, IR and NMR are shown below.

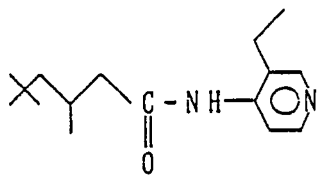

IR : 3350, 2900 to 3110, 1690, 1594

NMR: 0.93 (9H, s), 1.05 (3H, d), 1.28 (3H, t), 1.1 to 1.4 (2H, m), 2.0 to 2.3 (1H, m), 2.32 (2H, t), 2.62 (2H, g), 0.14 (1H, d), 0.37 (1H, s), 8.40 (1H, d)

Example 148.

Into a 100 ml three-necked flask were introduced 0.36 g (8.9 m moles) of 60% sodium hydride and 10 ml of dimethyl formamide and, under an atmosphere of nitrogen, a dimethyl formamide solution of 1.95 g (7.4 m moles) of N-(3-methyl-4-pyridyl)-3,5,5-trimethyl hexanoic acid amide and a dimethyl formamide solution of 1.27 g (8.9 m moles) of methyl iodide were added dropwise thereto. After agitation for 1.5 hours at room temperature, the reaction mixture was poured into ice water and extracted with ethyl acetate. The ethyl acetate layer was washed with a saturated aqueous solution of sodium chloride followed by drying over anhydrous sodium sulfate. The solvent was removed by distillation under reduced pressure followed by purification by the silica gel column chromatography to give 0.57 g (yield 28.0%) of oily N-methyl-N-(3-ethyl-4-pyridyl)-3,5,5-trimethyl hexanoic acid amide. The structural formula, IR and NMR are shown below.

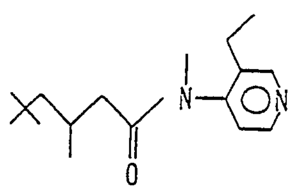

IR : 2900 to 3050, 1678, 1600, 1507

NMR: 0.84 (9H, s), 0.89 (3H, d), 0.8 to 1.1 (3H, m), 1.28 (3H, t), 1.9 to 2.2 (3H, m), 2.61 (2H, q), 3.20 (3H, t), 7.03 (1H, d), 8.53 (1H, d), 8.64 (1H, s)

Example 149.

Into a 100 ml three-necked flask were introduced 0.22 g (9.1 m moles) of magnesium and 10 ml of ethyl ether and, under an atmosphere of nitrogen, an ethyl ether solution of 1.88 g (9.1 m moles) of 3,5,5-trimethylhexyl bromide was added dropwise thereto and, after heating for 2.5 hours under reflux, a benzene solution of 1.0 g (7.6 m moles) of 3-ethyl-4-cyano pyridine was added dropwise at room temperature and heated for 8 hours under reflux. The reaction mixture was admixed with a 5% aqueous solution of ammonium chloride and extracted with ethyl acetate. After removal of the solvent by distillation under reduced pressure, 20 ml of a 6-normal aqueous solution of hydrochloric acid were added and heated for 2

hours under reflux. The aqueous layer obtained by the extraction of the reaction mixture with ethyl acetate was rendered alkaline with sodium carbonate followed by extraction with ethyl ether and washed with a saturated aqueous solution of sodium chloride followed by drying over anhydrous sodium sulfate. The solvent was removed by distillation under reduced pressure followed by purification by the silica gel column chromatography to give 0.20 g (yield 10.0%) of oily 3-ethyl-4-pyridyl 3,5,5-trimethylhexyl ketone. The structural formula, IR and NMR are shown below.

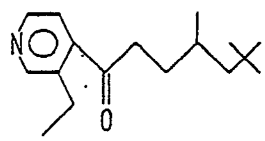

IR : 2900 to 3060, 1715, 1602, 1480

NMR: 0.84 (9H, s), 0.92 (3H, d), 1.23 (3H, t), 1.1 to 1.8 (5H, m), 2.5 to 3.0 (4H, m), 7.30 (1H, d), 8.56 (1H, d), 8.58 (1H, s)

Example 150.

Under an atmosphere of nitrogen, 0.25 g (6.5 m moles) of aluminum hydride was added to 10 ml of ethyl ether and, under agitation at room temperature, an ethyl ether solution of 1.28 g (4.9 m moles) of N-(3-ethyl-4-pyridyl)-3,5,5-trimethyl hexanoic acid amide was added dropwise. After heating for 3 hours under reflux, the reaction mixture was poured into ice water and the ethyl ether solution was washed with a saturated aqueous solution of sodium chloride followed by drying over anhydrous sodium sulfate. The solvent was removed by distillation under reduced pressure followed by purification by the silica gel column chromatography to give 0.34 g (yield 28%) of oily 3-ethyl-4-(3,5,5-trimethylhexylamino) pyridine. The structural formula, IR and NMR are shown below.

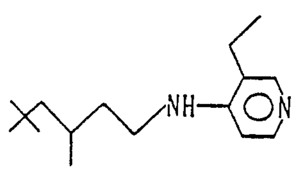

IR : 3300, 2900 to 3060, 1613, 1480

NMR: 0.91 (9H, s), 1.00 (3H, d), 1.24 (3H, t), 1.1 to 1.8 (3H, m), 2.45 (2H, q), 3.0 to 3.3 (2H, m), 6.44 (1H, d), 8.05 (1H, s), 8.17 (1H, d)

Examples 151 to 158.

The pyridine derivative obtained in the above described example was reacted with a specific acid shown in Table 19 to give corresponding pyridinium salts. The results are shown in Table 19 together with the physical properties.

Table 19

| Example | Compound | Kind of salt | Melting point (°C) |
|---|---|---|---|
| 151 | 19 | Oxalic acid | 82.0-83.6 |
| 152 | 19 | Hydrochloric acid | 172.6-173.7 |
| 153 | 19 | Phosphoric acid | 88.2-89.3 |
| 154 | 28 | Oxalic acid | 80.3-81.3 |
| 155 | 44 | Oxalic acid | 116.1-117.9 |
| 156 | 46 | Hydrochloric acid | decomposed at 175°C |
| 157 | 46 | p-Tolyl sulfonic acid | 90.3-91.0 |
| 158 | 52 | Oxalic acid | 83.8-84.7 |

Test Example.

A stalk of rice cut in a length of 10 cm was immersed for 1 minutes in a chemical solution of one of the compounds obtained in the examples (excepting the compound of Example 4) in a concentration of 500 ppm and, after air-drying, put into a test tube containing water, in which larvae (3-periods old) of tobiirounka were released and kept standing in a thermostatic chamber at 25 °C with a cotton plug.

After 7 days of the testing, life or death of the above mentioned larvae was checked to find 100% dead larvae with each of the compounds obtained in the examples. Comparative Test Example.

In place of the compounds used in Test Example, the same test was undertaken by using:

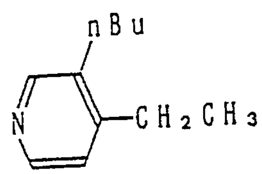

(CA 65, 10556f, 1966);

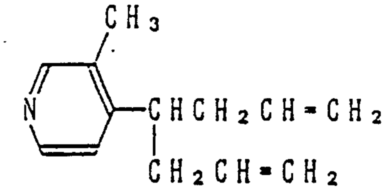

(CA 85, 32850y, 1976);

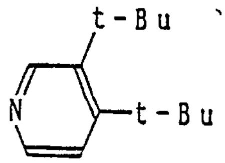

(CA 100, 155997n, 1984); or

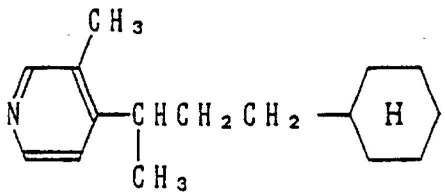

(CA 109, 6426j, 1988)

to find that the % dead larvae was 92%, 90%, 92% and 98%, respectively.

Industrial utilizability

The novel pyridine derivatives and salts thereof of the present invention exhibit strong insecticidal-acaricidal activity. Since they are readily decomposable, in addition, no problems are caused relative to retentiveness and accumulativeness. Since the structures are different from known chemicals, furthermore, the activity against pests is also different so that they can be used effectively for the control of the pests of which a resistive species has appeared. Accordingly, the present invention is useful for the elimination of the pests on agricultural crops, horticultural crops and the like, control of hygienic pests and the like.

## Claims

**1.** A pyridine derivative represented by the general formula (I)

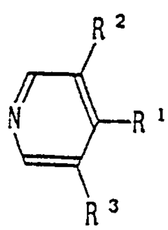

...... (I)

[in the formula, $R^1$ denotes a straight-chain or branched alkyl group of 2 to 20 carbons and the carbons of the alkyl chain in $R^1$ can be substituted with the following substituent groups at any position, in any number and in any combination where the direction of bonding is also not limitative,

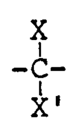

(in which X is a halogen and denotes a fluorine, chlorine, bromine or iodine and X' denotes a hydrogen or a halogen),

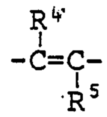

(in which $R^4$ and $R^5$ each denote a hydrogen or an alkyl group of 1 to 4 carbons),

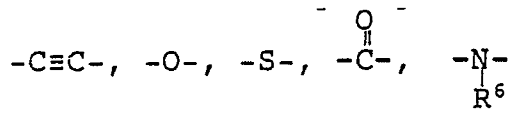

(in which $R^6$ denotes a hydrogen or an alkyl group of 1 to 4 carbons),

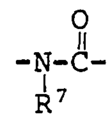

(in which $R^7$ denotes a hydrogen or an alkyl group of 1 to 4 carbons) and =N-O-. And, the hydrogen at the carbon terminal of $R^1$ can be replaced with the following substituent group. Cycloalkyl group of 3 to 16 carbons, cycloalkyl group of 3 to 16 carbons substituted by any number of alkyl, alkenyl, alkynyl groups of 1 to 6 carbons or halogens (incidentally, these cyclic rings can be a bicyclo ring or tricyclo ring), cycloalkenyl group of 3 to 16 carbons, cycloalkenyl group of 3 to 16 carbons substituted by any number of alkyl, alkenyl, alkynyl groups of 1 to 6 carbons or halogens (incidentally, these cyclic rings can be a bicyclo ring or tricyclo ring), cyclic ether and cyclic thioether (a plural number of ethers can be included), halogen or trihalomethyl group. $R^2$ denotes a straight-chain or branched alkyl group, alkenyl group or alkynyl group of 2 to 6 carbons, cycloalkyl group of 3 to 6 carbons or straight-chain or branched alkyl group, alkenyl group or alkynyl group of 1 to 6 carbons substituted with a cycloalkyl group of 3 to 6 carbons. Incidentally, however, the total number of carbons in $R_1$ is at least 4 and $R^1$ and $R^2$ are not the same ones. And, a part of $R^1$ and $R^2$ each can be a methylene group forming a cyclic structure. In this case, the ring has a size of 4 to 8 carbons. $R^3$ denotes a hydrogen or a straight-chain or branched alkyl group of 1 to 6 carbons.] and a salt thereof.

2. An insecticidal-acaricidal agent comprising, as an effective ingredient, a pyridine derivative represented by the general formula (I)

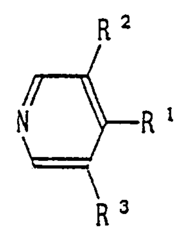

······ (I)

[in the formula, $R^1$ denotes a straight-chain or branched alkyl group of 2 to 20 carbons and the carbons of the alkyl chain in $R^1$ can be substituted with the following substituent groups at any position, in any number and in any combination where the direction of bonding is also not limitative,

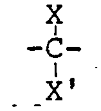

(in which X is a halogen and denotes a fluorine, chlorine, bromine or iodine and X' denotes a hydrogen or a halogen,

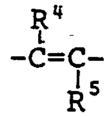

(in which $R^4$ and $R^5$ each denote a hydrogen or an alkyl group of 1 to 4 carbons)

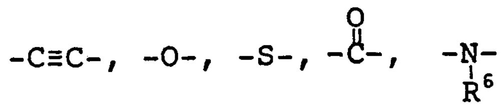

, (in which $R^6$ denotes a hydrogen or an alkyl group of 1 to 4 carbons),

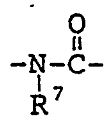

(in which $R^7$ denotes a hydrogen or an alkyl group of 1 to 4 carbons) and =N-O-. And, the hydrogen at the carbon terminal of $R^1$ can be replaced with the following substituent group. Cycloalkyl group of 3 to 16 carbons, cycloalkyl group of 3 to 16 carbons substituted by any number of alkyl, alkenyl, alkynyl groups of 1 to 6 carbons or halogens (incidentally, these cyclic rings can be a bicyclo ring or tricyclo ring), cycloalkenyl group of 3 to 16 carbons, cycloalkenyl group of 3 to 16 carbons substituted by any number of alkyl, alkenyl, alkynyl groups of 1 to 6 carbons or halogens (incidentally, these cyclic rings can be a bicyclo ring or tricyclo ring), cyclic ether and cyclic thioether (a plural number of ethers can be included), halogen or trihalomethyl group. $R^2$ denotes a straight-chain or branched alkyl group, alkenyl group or alkynyl group of 2 to 6 carbons, cycloalkyl group of 3 to 6 carbons or straight-chain or branched alkyl group, alkenyl group or alkynyl group of 1 to 6 carbons substituted with a cycloalkyl group of 3 to 6 carbons. Incidentally, however, the total number of carbons in $R_1$ is at least 4 and $R^1$ and $R^2$ are not the same ones. And, a part of $R^1$ and $R^2$ each can be a methylene group forming a cyclic structure. In this case, the ring has a size of 4 to 8 carbons. $R^3$ denotes a hydrogen or a straight-chain or brnached alkyl group of 1 to 6 carbons.] or a salt thereof.

# INTERNATIONAL SEARCH REPORT

International Application No PCT/JP90/00313

**I. CLASSIFICATION OF SUBJECT MATTER** (if several classification symbols apply, indicate all) [6]

According to International Patent Classification (IPC) or to both National Classification and IPC Int. $Cl^5$

C07D213/16, C07D213/26, C07D213/30, C07D213/32, C07D213/50, C07D213/53, C07D213/68, C07D213/74,C07D213/75,C07D217/02,C07D405/06,C07D409/06,A01N43/40

**II. FIELDS SEARCHED**

Minimum Documentation Searched [7]

| Classification System | Classification Symbols |
|---|---|
| IPC | C07D213/16,C07D213/26-40,C07D213/48-53,C07D213/56,C07213/68, C07D213/70,C07D213/72-75,C07D213/81,C07D217/02,C07D221/04, C07D405/06,C07D409/06,A01N43/40 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 63-30469 (Farmitalia Carlo Erba S.p.A.), 9 February 1988 (09. 02. 88), Scope of Claim & DK, A, 375987 & EP, A, 253681 & AU, A, 7569087 & HU, A, 44506 | 1 |
| A | JP, A, 59-148765 (Könlrutium Hür Electrochemisch Industry GmbH), 25 August 1984 (25. 08. 84), Scope of Claim, & EP, A, 116122 | 1 |
| A | JP, A, 58-109472 (Univablot), 29 June 1983 (29. 06. 83), Scope of Claim & FR, B1, 2518089 & US, A, 4477453 | 1 |
| A | JP, B1, 46-16106 (Sankyo Co., Ltd.), 1 May 1971 (01. 05. 71), Scope of Claim (Family: none) | 2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 28, 1990 (28. 05. 90) | June 11, 1990 (11. 06. 90) |
| **International Searching Authority** | **Signature of Authorized Officer** |
| Japanese Patent Office | |